# EUROPEAN PATENT APPLICATION

(11) **EP 4 563 701 A1**
(43) Date of publication of application: **04.06.2025**
(21) Application number: 23846696.5
(22) Date of filing: 28.07.2023
(51) Int. Cl.: C12N 15/52, C12N 9/00, C12N 15/11, C12N 15/66, C40B 40/08

(54) **PEPTIDE SYNTHETASE LIBRARY CONSTRUCTION METHOD**

(30) Priority: 29.07.2022 JP 2022121822
(71) Applicant: National University Corporation Kobe University, Kobe-shi, Hyogo 657-8501 (JP)
(72) Inventor: TSUGE, Kenji, Kobe-shi, Hyogo 657-8501 (JP); KONDO, Akihiko, Kobe-shi, Hyogo 657-8501 (JP); VARADA, Jagadeesh, Kobe-shi, Hyogo 657-8501 (JP)
(74) Representative: Vossius & Partner Patentanwälte Rechtsanwälte mbB
(86) International application number: PCT/JP2023/027837
(87) International publication number: WO 2024/024966

(57) **Abstract**

The present disclosure pertains to produce a novel NRPS. **In** the present disclosure, a restriction enzyme recognition sequence was successfully introduced into a nucleic acid encoding an NRPS while maintaining a function of producing a target NRP. Various NRPSs can be produced using the introduced restriction enzyme recognition sequence. **In** one aspect, the present disclosure provides a nucleic acid containing a non-native restriction enzyme recognition sequence and encoding an NRPS. **In** another aspect, the present disclosure provides a method for efficiently producing a nucleic acid encoding a novel NRPS using a non-native restriction enzyme recognition sequence. **In** addition, the present disclosure also provides a novel NRPS and a novel NRP.

## Description

### Technical Field

The present disclosure provides a modified non-ribosomal peptide synthetase (NRPS) and a use thereof. The present disclosure also provides a plasmid or plasmid library encoding a modified NRPS, or a method for using or producing the same. The present disclosure also provides a non-ribosomal peptide (NRP) produced using an NRPS.

### Background Art

An NRPS is known as an enzyme that synthesizes a peptide having a specific structure (Non Patent Literature 1). Since peptide synthesis by an NRPS does not depend on a ribosome, it is known that synthesis of a peptide containing a non-proteinaceous amino acid (ornithine, D-form amino acid, or the like) is possible, and there are 800 or more types of amino acids that can be contained in an NRP. An NRP synthesized by an NRPS is used in various fields such as antibiotics or pesticides. It is desired to develop a novel NRPS so as to efficiently produce an NRP and to provide a novel NRP.

### Citation List

### Non Patent Literature

Non Patent Literature 1: Angew Chem Int Ed Engl. 2017 Mar 27; 56 (14): 3770-3821.

### Summary of Invention

### Solution to Problem

The present inventors have succeeded in introducing a restriction enzyme recognition sequence into a nucleic acid encoding an NRPS while maintaining a function of producing a target NRP. Various NRPSs can be produced using the introduced restriction enzyme recognition sequence. In one aspect, the present disclosure provides a nucleic acid containing a non-native restriction enzyme recognition sequence and encoding an NRPS. In another aspect, the present disclosure provides a method for efficiently producing a nucleic acid encoding a novel NRPS using a non-native restriction enzyme recognition sequence. In addition, the present disclosure also provides a novel NRPS and a novel NRP.

Accordingly, the present disclosure provides the following.

### (Item 1)

A method for producing a product plasmid containing a nucleic acid sequence encoding a non-ribosomal peptide synthetase (NRPS), the method including:
a step of preparing a set of starting plasmids, each of the set of starting plasmids containing a base sequence encoding at least one NRPS module and at least one restriction enzyme recognition sequence;
a step of preparing a mixture of containing a plurality of resulting cleaved nucleic acid fragments by treating the set of starting plasmids with a restriction enzyme;
a step of forming a linked nucleic acid by ligating the plurality of cleaved nucleic acid fragments; and
a step of bringing the linked nucleic acid into contact with a transgenic organism to form a product plasmid.

### (Item 2)

The method according to the preceding item, in which the plurality of cleaved nucleic acid fragments include a set of cleaved nucleic acid fragments containing the same overhang sequence and containing a base sequence encoding a plurality of types of NRPS modules.

### (Item 3)

The method according to any one of the preceding items, in which the plurality of cleaved nucleic acid fragments include a set of cleaved nucleic acid fragments containing the same overhang sequence and containing the base sequence encoding an NRPS module capturing a plurality of types of amino acids.

### (Item 4)

The method according to any one of the preceding items, in which each of the cleaved nucleic acid fragments contains an overhang sequence of 3 to 5 bases in length.

### (Item 5)

The method according to any one of the preceding items, in which in the starting plasmid, the restriction enzyme recognition sequence is located between base sequences encoding adjacent NRPS modules.

### (Item 6)

The method according to any one of the preceding items, in which in the starting plasmid, the restriction enzyme recognition sequence is located between a C domain coding region and an A domain coding region of the NRPS module.

### (Item 7)

The method according to any one of the preceding items, in which in the starting plasmid, the restriction enzyme recognition sequence is located in a region between the C domain coding region and the A domain coding region of the NRPS module, and the region does not include the C domain coding region and the A domain coding region.

### (Item 8)

The method according to any one of the preceding items, in which the at least one restriction enzyme recognition sequence is present in the base sequence encoding an NRPS module.

### (Item 9)

The method according to any one of the preceding items, in which the at least one restriction enzyme recognition sequence is present outside the base sequence encoding an NRPS module.

### (Item 10)

The method according to any one of the preceding items, in which the restriction enzyme recognition sequence is a non-native sequence in the NRPS module.

### (Item 11)

The method according to any one of the preceding items, in which each of the cleaved nucleic acid fragments contains at most one NRPS module.

### (Item 12)

The method according to any one of the preceding items, in which the restriction enzyme recognition sequence is cleaved at a site different from a restriction enzyme-specific recognition site.

### (Item 13)

The method according to any one of the preceding items, in which the restriction enzyme recognition sequence includes a recognition sequence for SfiI, BglI, AlwNI, DraIII, PflMI, BstAPI, AarI, BbsI, BsaI, BsmBI, or BspQI.

### (Item 14)

The method according to any one of the preceding items, in which each of the starting plasmids contains a base sequence encoding 4 to 12 NRPS modules.

### (Item 15)

The method according to any one of the preceding items, in which in the step of preparing the mixture containing the cleaved nucleic acid fragments, each of the starting plasmids produces cleaved nucleic acid fragments containing overhang sequences that are different from one another.

### (Item 16)

The method according to any one of the preceding items, in which the mixture containing the cleaved nucleic acid fragments is a solution.

### (Item 17)

The method according to any one of the preceding items, in which each of the starting plasmids independently contains the same or different selectable marker sequences.

### (Item 18)

The method according to any one of the preceding items, in which the selectable marker sequence includes a drug-resistant marker sequence.

### (Item 19)

The method according to any one of the preceding items, in which each of the starting plasmids independently contains the same or different origins of replication operable in Bacillus subtilis.

### (Item 20)

The method according to any one of the preceding items, in which each of the starting plasmids independently contains the same or different promoter regions upstream of the base sequence encoding an NRPS.

### (Item 21)

A product plasmid or plasmid library produced by the method according to any one of the preceding items.

### (Item 22)

An NRPS produced using the product plasmid or plasmid library according to any one of the preceding items.

### (Item 23)

An NRP produced using the NRPS according to any one of the preceding items.

### (Item 24)

A nucleic acid encoding a non-ribosomal peptide synthetase (NRPS), the nucleic acid containing:
a base sequence encoding at least one NRPS module; and
a non-native restriction enzyme recognition sequence for the NRPS module.

### (Item 25)

The nucleic acid according to any one of the preceding items, in which the restriction enzyme recognition sequence is located between the base sequences encoding adjacent NRPS modules.

### (Item 26)

The nucleic acid according to any one of the preceding items, in which the restriction enzyme recognition sequence is located between a C domain coding region and an A domain coding region of the NRPS module.

### (Item 27)

The nucleic acid according to any one of the preceding items, in which the restriction enzyme recognition sequence is located in a region between the C domain coding region and the A domain coding region of the NRPS module, and the region does not include the C domain coding region and the A domain coding region.

### (Item 28)

The nucleic acid according to any one of the preceding items, in which the restriction enzyme recognition sequence is a recognition sequence for a restriction enzyme that recognizes a sequence containing a region in which any type of base is present.

### (Item 29)

The nucleic acid according to any one of the preceding items, in which the restriction enzyme recognition sequence is a recognition sequence for SfiI.

### (Item 30)

The nucleic acid according to any one of the preceding items, in which the number of NRPS modules encoded by the nucleic acid is 4 to 12.

### (Item 31)

A method for producing a product nucleic acid containing a nucleic acid sequence encoding a non-ribosomal peptide synthetase (NRPS), the method including:
a step of preparing the nucleic acid according to any one of the preceding items as a starting nucleic acid;
a step of preparing a cleaved nucleic acid fragment by treating the nucleic acid with a restriction enzyme that recognizes the restriction enzyme recognition sequence; and
a step of forming a product nucleic acid that is different from the starting nucleic acid by ligating the cleaved nucleic acid fragments.

### (Item 32)

A product nucleic acid produced by the method according to any one of the preceding items.

In the present disclosure, it is intended that the one or the plurality of features can be provided in further combination in addition to the specified combination. Still further embodiments and advantages of the present disclosure will be appreciated by those skilled in the art upon reading and understanding the following detailed description, if necessary. Advantageous Effects of Invention

According to the present disclosure, it is possible to efficiently produce a novel NRPS, thereby providing an NRPS that enables efficient NRP production or an NRPS that provides a novel NRP.

### Brief Description of Drawings

FIG. 1 illustrates an outline of a structure of plipastatin (A) and NRPS (B) that produces the same. A: adenylation domain, T: thiolation domain, C: condensation domain, TE: thioesterase domain, E: epimerization domain.
FIG. 2 illustrates an alignment of a C-A linker region between plipastatin NRPS modules and a SfiI recognition sequence introduction site (square box portion).
FIG. 3 illustrates an outline of introduction of a SfiI recognition sequence.
FIG. 4 illustrates an outline of assembly of unit DNAs by the OGAB method.
FIG. 5 illustrates a comparison of gel electrophoresis bands when plasmids ppsW03 and ppsW10 are treated with various restriction enzymes.
FIG. 6 illustrates HPLC analysis results of extracts of culture solutions of respective strains. The results of strains (a) BEST8628, (b) ppsW03/BUSY9261, (c) ppsW10/BUSY9261, and (d) BUSY9261 are shown, respectively. The vertical axis represents an absorbance at 205 nm, and the horizontal axis represents a retention time (minutes).
FIG. 7 illustrates measurement results of substances produced by ppsW03/BUSY9621 strain by a mass spectrometer. a to d represent peaks of plipastatin.
FIG. 8 illustrates the amount of plipastatin produced of each strain. The results of BEST8628, ppsW10/BUSY9261, and ppsW03/BUSY9261 strains are shown from the left. The vertical axis represents the amount (µg/mL) of plipastatin produced, and the horizontal axis represents a culture time (hours).
FIG. 9 illustrates an outline of a chimeric NRPS gene.
FIG. 10A illustrates MS/MS analysis results of a novel chimeric peptide found from BUSY9261/chmera 1. The results of chimeric peptide 1 are shown.
FIG. 10B illustrates MS/MS analysis results of a novel chimeric peptide found from BUSY9261/chmera 2. The results of chimeric peptide 2 are shown.
FIG. 10C illustrates MS/MS analysis results of a novel chimeric peptide found from BUSY9261/chmera 3. The results of chimeric peptide 3 are shown.
FIG. 11 illustrates an outline of introduction of a SfiI recognition sequence into a surfactin synthetase.
FIG. 12 illustrates an outline of construction of various NRPSs by the Combi-OGAB method.

### Best Mode for Carrying Out the Invention

Hereinafter, the present disclosure will be described while showing the best mode. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used in the present specification are to be understood as being used in the sense commonly used in the art unless otherwise stated. Therefore, unless defined otherwise, all technical and scientific terms used in the present specification have the same meaning as commonly understood by those skilled in the art to which the present disclosure pertains. In the case of conflict, the present specification (including definitions) will control.

Hereinafter, the definitions of the terms and/or basic technical concepts that are particularly used in the present specification will be described as appropriate.

As used herein, the "non-ribosomal peptide synthetase" (in the present specification, also referred to as an NRPS) refers to a protein that synthesizes a peptide (which can be referred to as a non-ribosomal peptide (NRP)) using an amino acid as a substrate without the need for mRNA. Usually, a plurality of molecules of the NRPS protein form a complex to produce a peptide, but in the present specification, the NRPS may not form a protein complex. The NRPS molecule may bind to another NRPS via a COM domain located at the end to form an NRPS protein complex. Besides 20 types of essential amino acids, nonproteinaceous amino acids such as ornithine are used as substrates for the NRPS. Peptides and cyclic or branched peptides with non-amino acid modifications such as fatty acids or sugar chains can also be synthesized by the NRPS. The NRPS is known to be responsible for the synthesis of peptides such as cyclosporine and vancomycin in microorganisms. Typically, NRPSs in organisms such as the genus Bacillus (for example, B. subtilis, B. amyloliquefaciens, and B. licheniformis), Brevibacillus brevis, Paenibacillus polymyxa, Photorhabdus asymbiotica, Photorhabdus luminescens, the genus Xenorhabdus (for example, X. bovienii, X. budapestensis, X. ducetiae, X. indica, X. miraniensis, X. nematophila, X. stockiae, and X. szentirmaii), the genus Streptomyces (for example, S. verticillus and S. roseosporus), the genus Penicillium, the genus Aspergillus, the genus Tolypocladium (for example, T. inflatum and T. niveum), the genus Fusarium, the genus Microcystis, and the like are known.

In one embodiment, a protein can be determined to be an NRPS by the protein containing one or a plurality of motifs selected from the group consisting of an A1 core motif sequence (L(TS)YxEL: SEQ ID NO: 44), an A2 core motif sequence (LKAGxAYL(VL)P(LI)D: SEQ ID NO: 45), an A3 core motif sequence (LAYxxYTSG(ST)TGxPKG: SEQ ID NO: 46), an A4 core motif sequence (FDxS), an A5 core motif sequence (NxYGPTE: SEQ ID NO: 47), an A6 core motif sequence (GELxIxGxG(VL)ARGYL: SEQ ID NO: 48), an A7 core motif sequence (Y(RK)TGDL: SEQ ID NO: 49), an A8 core motif sequence (GRxDxQVKIRGxRIELGEIE: SEQ ID NO: 50), an A9 core motif sequence (LPxYM(IV)P: SEQ ID NO: 51), an A10 core motif sequence (NGK(VL)DR: SEQ ID NO: 52), a T core motif sequence (DxFFxxLGG(HD)S(LI): SEQ ID NO: 53), a C1 core motif sequence (SxAQxR(LM)(WY)xL: SEQ ID NO: 54), a C2 core motif sequence (RHExLRTxF: SEQ ID NO: 55), a C3 core motif sequence (MHHxISDG(WV)S: SEQ ID NO: 56), a C4 core motif sequence (YxD(FY)AVW: SEQ ID NO: 57), a C5 core motif sequence ((IV)GxFVNT(QL)(CA)xR: SEQ ID NO: 58), a C6 core motif sequence ((HN)QD(YV)PFE: SEQ ID NO: 59), and a C7 core motif sequence (RDxSRNPL: SEQ ID NO: 60). Preferably, the NRPS may contain consecutive (for example, A8 to A10 core motif sequences) motif sequences among the A1 to A10 core motif sequences, the T core motif sequence, and the C1 to C7 core motif sequences in the same order. When a plurality of motif sequences are contained, 1 to 3 mutations (for example, conservative amino acid substitutions) may be contained in the entire motif sequence.

As used herein, the "non-ribosomal peptide (NRP)" refers to a peptide that can be produced or has been produced by an NRPS, and also includes a peptide (consisting of natural 20 amino acids) that can be synthesized by ribosomes. Typically, an NRP is a peptide that cannot be synthesized by ribosomes.

As used herein, the "NRPS module" is a partial structure of an NRPS, and in principle, one NRPS module is involved in binding of one amino acid in an NRPS, but may also be involved in binding of a substrate other than an amino acid (β-amino fatty acid, β-hydroxy fatty acid, polyketide, or the like). Typically, one NRPS module contains one A domain, one T domain, and one C domain, and may contain other domains such as TE. In a case where a middle portion of a region encoding any of the A, T, and C domains is located at an end of a nucleic acid molecule fragment, when a region encoding at least a part of each of the A, T, and C domains is present on one nucleic acid molecule fragment, the nucleic acid molecule fragment contains one NRPS module. For example, one NRPS module contains, from the N-terminus to the C-terminus, (A, T, and C), (T, C, and A), or (C, A, and T) domains. Typically, in a case where a plurality of NRPS modules are encoded on one molecule of nucleic acid, the largest number of regions containing the A, T, and C domains from the N-terminus to the C-terminus in the same order are counted as one NRPS module (for example, for a nucleic acid encoding a domain of A-T-C-A-T-C-A-T-C, when T-C-A or C-A-T is counted as one NRPS module, there are two NRPS modules, but when A-T-C is counted as one NRPS module, there are three NRPS modules; thus, the number of NRPS modules in this case is 3). As used herein, typically, one NRPS module is disposed between two restriction enzyme recognition regions. A region containing only one or two domains of A, T, and C may be referred to as an incomplete NRPS module.

As used herein, the "A domain" is an adenylation domain of an NRPS, and is considered to capture a specific amino acid and activate the amino acid by ATP or the like. The A domain has a size of about 500 to 550 amino acids and is considered to have substrate specificity for specific amino acids. The A domain is considered to have substrate specificity for specific amino acids in an A_{core} subdomain of about 400 amino acids with an amino acid binding pocket and an A_{sub} subdomain with lysine involved in catalytic activity. In one embodiment, the A domain may refer to a region from 70 amino acids upstream of the A1 core motif sequence (L(TS)YxEL) to 40 amino acids downstream of the A10 core motif sequence (NGK(VL)DR).

As used herein, the "T domain" (also referred to as a PCP domain) is a thiolated domain of an NRPS, in which (the thiol portion of the cysteine residue of) the domain is considered to capture amino acids received from the A domain. The T domain is considered to have a size of about 70 to 90 amino acids. In one embodiment, the T domain may refer to a region from 20 amino acids upstream to 80 amino acids downstream of the T core motif sequence (DxFFxxLGG(HD)S(LI)).

As used herein, the "C domain" is a condensation domain of an NRPS, and is considered to have a function of binding a peptide or amino acid bound to a previous NRPS module to an amino acid captured in the T domain. The C domain is considered to have a size of about 450 amino acids. In one embodiment, the C domain may refer to a region from 20 amino acids upstream of the C1 core motif sequence (SxAQxR(LM)(WY)xL) to 70 amino acids downstream of the C7 core motif sequence (RDxSRNPL).

As used herein, the "TE domain" is a thioesterase domain of an NRPS, and is considered to have a function of releasing an NPR from the NRPS. The TE domain may have a function of circularizing the NPR.

As used herein, the "linker region" refers to a short (for example, 20 amino acids or less in length) region that is present between NRPS modules or between domains of the NRPS. Since the linker region is present between the extensions of the modules and domains, the linker region can be determined by determining the extensions of these modules and domains.

As used herein, the description of "between" two regions (for example, nucleic acid regions encoding a domain) includes the two regions themselves. Typically, between two regions refers to a region from a bond that divides the number of amino acids or nucleotide residues (N) constituting one region by two (N is an even number) or an amino acid or nucleotide residue (N is an odd number) to a bond that divides the number of amino acids or nucleotide residues (M) constituting another region by two (M is an even number) or an amino acid or nucleotide residue (M is an odd number).

As used herein, the description of "boundary" of two regions (for example, nucleic acid regions encoding a domain) refers to a bond between two amino acid residues that are adjacent to each other in a peptide or a bond between two nucleotides that are adjacent to each other in a polynucleotide, which is present between the two regions. In one embodiment, a "boundary" of two regions is present between the two regions and not within the two regions.

The "non-native" base sequence refers to a region that contains a base sequence that encodes a different amino acid region when the peptide encoded by the base sequence (such as the NRPS module) is compared to the native corresponding peptide exhibiting the highest identity thereto (such as the NRPS module).

The "restriction enzyme recognition sequence" refers to a base sequence specific for each type of restriction enzyme and necessary for cleavage of a nucleic acid by a restriction enzyme. The restriction enzyme recognition sequence may include a portion designating any base (typically, denoted by "N" in a motif). In particular, a portion of the restriction enzyme recognition sequence required to be any one of G, C, T, or A may be referred to as a restriction enzyme recognition specific site. For example, SfiI (motif 5'-GGCCNNNN/NGGCC-3') cleaves a site between two restriction enzyme specific recognition sites, and AarI (motif 5'-CACCTGCNNNN/NNNN-3') cleaves a site distant from the restriction enzyme-specific recognition sites.

As used as used herein, the "plasmid" refers to a circular DNA that is present separately from chromosomes in a cell or that is present separately from chromosomes when introduced into a cell.

As used herein, the "nucleic acid sequence that promotes plasmid replication" refers to any nucleic acid sequence that promotes replication (in this context, it is the same as amplification) of a plasmid present in a host cell (for example, Bacillus subtilis) when introduced into the host cell. Preferably, the nucleic acid sequence that promotes plasmid replication is operably linked to, but not limited to, a nucleic acid sequence encoding a target plasmid. Details of the nucleic acid sequence that promotes plasmid replication, the target plasmid, the relationship therebetween, and the like, are described elsewhere in the present specification. Examples of the nucleic acid sequence that promotes plasmid replication include a nucleic acid sequence containing an origin of replication that operates in a target host cell (for example, Bacillus subtilis). For example, a nucleic acid sequence that promotes plasmid replication in Bacillus subtilis has an ability to promote plasmid replication in Bacillus subtilis, and may further have an ability to promote plasmid replication in microorganisms other than Bacillus subtilis (for example, E. coli). A nucleic acid sequence that promotes plasmid replication in Bacillus subtilis and other organisms (for example, E. coli) may be a nucleic acid sequence in which the same region is utilized to promote plasmid replication in both Bacillus subtilis and other organisms (for example, E. coli), or may be a sequence located at a distance. For example, it has been shown that plasmid pSTK1 (Issay Narumt et al., BIOTECHNOLOGY LETTERS, Volume 17 No.5 (May 1995) pp. 475-480) and plasmid pBS195 (Molekuliarnaia Genetika et al., 01 May 1991, (5): 26-29, PMID: 1896058) are capable of replicating in the same replication initiation region in both Bacillus subtilis and E. coli, and such nucleic acid sequences may be used as nucleic acid sequences that promote plasmid replication in both Bacillus subtilis and E. coli. In addition, a shuttle plasmid (P Trieu-Cuot et al., EMBO J. 1985 Dec 16; 4 (13A): 3583-3587) produced by linking BR322 (E. coli plasmid) and pC194 (Bacillus subtilis plasmid), and a plasmid of B. subtilis Secretory Protein Expression System containing pUBori and ColE1 ori provided by Takara Bio Inc. (Shiga prefecture) are replicable in both E. coli and Bacillus subtilis, and such distantly located nucleic acid sequences can also be used as nucleic acid sequences that promote plasmid replication in both Bacillus subtilis and E. coli.

Examples of a replication mechanism of nucleic acid (plasmid) in Bacillus subtilis include rolling circle type, theta type, oriC, and phage, and any mechanism can be used as a sequence to be replicated in Bacillus subtilis. The rolling circle type replication mechanism is a mechanism in which one strand of a double-stranded DNA is replicated and then the other strand is replicated, and the plasmid tends to be destabilized because the time during which the nucleic acid exists as a single-stranded DNA is long. The theta type replication mechanism is a mechanism in which replication of a double-stranded DNA (plasmid) is simultaneously initiated in two directions from an origin of replication as in the case of replication of a bacterial chromosome, and can be preferably used in replication of a long DNA (for example, 10 kb or more) as in the present disclosure (Janniere, L., A. Gruss, and S. D. Ehrlich. 1993. Plasmids, p. 625-644. InA. L. Sonenshein, J. A. Hoch, and R. Losick (ed.), Bacillus subtilis and othergram-positive bacteria: biochemistry, physiology and molecular genetics. American Society for Microbiology, Washington, D.C.). The oriC replication mechanism operates in the same manner as in replication of a host bacterial (for example, Bacillus subtilis) chromosome. Examples of the nucleic acid sequence that promotes plasmid replication in Bacillus subtilis include a plasmid or a portion thereof, or an origin of replication or a variant thereof, which is known to activate the replication mechanisms such as rolling circle type, theta type, oriC, and phage. As a rolling circle type plasmid, pUB110, pC194, pE194, pT181 and the like are known, and as a theta type plasmid, pAMβ1, pTB19, pLS32, pLS20, and the like are known.

The nucleic acid sequence that promotes plasmid replication in Bacillus subtilis may contain a sequence that is identical or similar to a known origin of replication (for example, 90% or more, 95% or more, 97% or more, 98% or more, 99% or more, or 99.5% or more sequence identity). For example, in a case where a DNA fragment in which a candidate DNA fragment and a selectable marker gene effective in Bacillus subtilis, such as a drug-resistant gene, are linked is introduced into Bacillus subtilis and then cultured (with addition of a drug or the like), and then extrachromosomal replication is performed, it can be determined that the candidate DNA fragment contains a nucleic acid sequence that promotes plasmid replication in Bacillus subtilis. A nucleic acid sequence that promotes plasmid amplification is a DNA fragment containing an origin of replication, and refers to a DNA fragment that can replicate independently of chromosomal DNA. Whether or not these DNA fragments can be replicated can be confirmed by a method in which a selectable marker gene such as a drug-resistant gene is linked to these DNA fragments, the resulting product is cultured under selective conditions such as a drug, plasmid DNA is then purified, and a band of the DNA is observed by electrophoresis. In addition to the origin of replication, the plasmid may contain a segregation mechanism gene, a selectable marker gene, and the like for ensuring segregation of the plasmid into a daughter cell.

As used herein, the "origin of replication" refers to a segment of a nucleic acid sequence to which a protein (for example, initiator DnaA protein or the like) that recognizes the nucleic acid sequence binds or to which RNA is synthesized to partially unwind a DNA double helix, and from which replication is initiated.

As used herein, the "transgenic organism" refers to an organism that can be used in the OGAB method and can incorporate an acyclic long chain nucleic acid to produce a plasmid. As the transgenic organism, a microorganism that spontaneously incorporates a nucleic acid can be used. A representative transgenic organism is Bacillus subtilis.

As used herein, the "Bacillus subtilis" refers to any non-pathogenic bacterium with a natural transformation ability, having the scientific name of Bacillus subtilis or including the genus Bacillus, that is closely related thereto, such as Bacillus amyloliquefaciens, Bacillus licheniformis, or Bacillus pumilus, which is considered to be an aerobic, gram-positive, and catalase-positive bacterium that is generally present in soil and plants and is present in the gastrointestinal tract of ruminants and humans, has a size of 0.7 to 0.8 × 2 to 3 µm, is mesophilic, has an optimal growth temperature of 25 to 40°C, and forms spores. In a preferred embodiment, Bacillus subtilis is Marburg 168 strain or its derivative strain RM125, which is a strain with high natural transforming potential among Bacillus subtilis. 168 strain is the most genetically studied gram-positive bacterium and is available from the American Type Culture Collection (ATCC) and the like together with RM125 strain, and can be suitably used in the present disclosure because it is itself harmless to humans and animals and is known to be applicable to the OGAB method.

As used herein, the "repeat sequence" or "tandem repeat" (a nucleic acid sequence) is a generic term for a nucleic acid sequence of an organism genome in which the same sequence is repeatedly (particularly several times or more) found. Any repeat sequence used in the art can be used in the present disclosure. Typically, a promoter sequence appears repeatedly.

As used as used herein, the "host cell" refers to a cell (including the progeny of such a cell) into which an exogenous nucleic acid, protein, or virus is introduced.

As used herein, the "protein", "polypeptide", "oligopeptide", and "peptide" are used in the same meaning as used herein, and refer to an amino acid polymer of any length. The polymer may be linear, branched, or cyclic. An amino acid may be a natural, non-native, or modified amino acid. The term also encompasses a natural or artificially modified polymer. Examples of such a modification include disulfide bond formation, glycosylation, lipidation, acetylation, phosphorylation, and any other manipulation or modification (for example, conjugation with a labeling component).

As used herein, the "polynucleotide" and "nucleic acid" are used to have the same meaning, and refer to a polymer of nucleotides of any length. Examples of the nucleic acid include DNA, RNA, cDNA, mRNA, rRNA, tRNA, microRNA (miRNA), and lncRNA. The term also includes a "polynucleotide derivative". The "polynucleotide derivative" refers to a polynucleotide containing a nucleotide derivative or having an unusual bond between nucleotides. The "nucleotide derivative" refers to a nucleotide having a structure different from a normal nucleotide used in natural DNA or RNA, and examples thereof include a locked nucleic acid (LNA), an ethylene nucleic acid such as a 2'-O,4'-C-ethylene bridged nucleic acid (ENA), other bridged nucleic acids (BNAs), a hexitol nucleic acid (HNA), an amido-bridged nucleic acid (AmNA), a morpholino nucleic acid, tricyclo-DNA (tcDNA), a polyether nucleic acid (see, for example, US 5,908,845), and a cyclohexene nucleic acid (CeNA). Examples of the unusual bond between nucleotides include a bond between oligonucleotides in which a phosphate diester bond is converted to a phosphorothioate bond, a bond between oligonucleotides in which a phosphate diester bond is converted to an N3'-P5' phosphoramidate bond, and a bond between oligonucleotides in which ribose and a phosphate diester bond are converted to peptide nucleic acid bonds.

Unless otherwise indicated, a particular nucleic acid sequence is also intended to encompass a conservatively modified variant (for example, condensed codon substituent) and complementary sequence thereof, as well as the sequence explicitly indicated. Specifically, the condensed codon substituent can be achieved by generating a sequence in which the third position of one or more selected (or all) codons is substituted with a mixed base and/or deoxyinosine residue. For example, a variant based on a particular wild-type sequence, such as an A domain of a particular NRPS, also contains a nucleic acid containing a sequence having at least 70%, at least 80%, at least 85%, at least 90%, at least 95%, at least 96%, at least 97%, at least 98%, at least 99%, or at least 99.5% sequence identity to the original sequence.

As used herein, the "gene" refers to a nucleic acid moiety that performs a certain biological function. The biological function includes coding for polypeptides or proteins, coding for protein non-coding functional RNAs (rRNA, tRNA, microRNA (miRNA), lncRNA, and the like), specifically binding to specific proteins, and controlling cleavage or replication of nucleic acids. Therefore, as used herein, the gene contains a promoter, a terminator, an enhancer, an insulator, a silencer, and an origin of replication, in addition to a nucleic acid moiety encoding a protein or a protein non-coding functional RNA. As used herein, the "gene product" may refer to a polypeptide, a protein, or a protein non-coding functional RNA encoded by a gene.

As used herein, the "deficient" of a gene refers to that a nucleic acid does not contain the gene or contains a gene that is modified so as not to perform the normal function (for example, the function of producing a functional protein) of the gene.

As used herein, the "operably linked" refers to placing the expression (actuation) of a desired sequence under the control of a transcriptional and translational regulatory sequence (for example, a promoter, an enhancer, or the like) or a translational regulatory sequence. In order for a promoter to be operably linked to a gene, the promoter is usually located immediately upstream of the gene, but is not necessarily located adjacent to the gene.

As used herein, the "homology" of nucleic acids refers to a degree of identity of two or more nucleic acid sequences with respect to one another, and having "homology" generally refers to having a high degree of identity or similarity. Therefore, the identity or similarity of the sequences is higher as homology of two nucleic acids is high. The "similarity" is a numerical value calculated for a similar base in addition to identity, and here, the similar bases refer to a case where some bases in a mixed base (for example, R = A + G, M = A + C, W = A + T, S = C + G, Y = C + T, K = G + T, H = A + T + C, B = G + T + C, D = G + A+ T, V = A + C + G, and N = A+ C + G + T) are identical. Whether two types of nucleic acids have homology can be determined by a direct comparison of sequences or a hybridization method under stringent conditions. When two nucleic acid sequences are directly compared, the genes have homology when the genes are typically at least 50% identical, preferably at least 70% identical, and more preferably, at least 80%, 90%, 95%, 96%, 97%, 98%, or 99% identical between the nucleic acid sequences.

Amino acids may be mentioned in the present specification by either their commonly known three letter symbols or their one character symbols recommended by the IUPAC-IUB Biochemical Nomenclature Commission. Similarly, nucleotides may be mentioned by their commonly recognized one character codes. As used herein, a comparison of similarity, identity, and homology of an amino acid sequence and a base sequence is calculated by using a sequence analysis tool BLAST and default parameters. For example, identity can be searched using BLAST 2.10.1+ (published on June 18, 2020) of the NCBI. As used herein, a value for identity generally refers to a value obtained when aligned under the default conditions using BLAST described above. However, when a higher value is obtained by changing a parameter, the highest value is set as the value of identity. **In** a case where identity is evaluated in a plurality of regions, the highest value in the plurality of regions is set as the value of identity. Similarity is a numerical value calculated by taking into consideration a similar amino acid in addition to identity.

As used herein, the "conservative amino acid substitution" refers to substitution of an amino acid in a peptide with an amino acid similar in another property, and the property of the peptide can be predicted to be similar before and after the conservative substitution. In one embodiment, the conservative amino acid substitution is substitution in an amino acid in the following group of amino acids with another amino acid in the same group.
- Group 1: glycine, alanine, valine, leucine, and isoleucine
- Group 2: serine and threonine
- Group 3: phenylalanine, tyrosine, and tryptophan
- Group 4: lysine, hydroxylysine, arginine, and histidine
- Group 5: cysteine, methionine, methionine sulfoxide, and homocysteine
- Group 6: proline and hydroxyproline
- Group 7: glutamic acid and aspartic acid
- Group 8: glutamine and asparagine

As used herein, unless otherwise specified, it is understood that reference to a biological material (for example, a protein, a nucleic acid, or a gene) is also a reference to a variant of the biological material (for example, a variant having a modification in an amino acid sequence) that performs a function similar to (but not to the same extent as) the biological function of the biological material. Such a variant can contain a fragment of the original molecule, and a molecule that is at least 30%, 40%, 50%, 60%, 70%, 80%, 90%, 95%, 98%, or 99% identical as compared to the sequence of the original molecule that is aligned over the amino acid sequence or nucleic acid sequence of the original biological material of the same size, or aligned by computer homology programs known in the art. A variant can contain a molecule with a modified amino acid (modification by, for example, disulfide bond formation, glycosylation, lipidation, acetylation, or phosphorylation) or a modified nucleotide (for example, modification by methylation).

As used herein, the "stringent conditions" refer to well-known conditions commonly used in the art. As the stringent conditions, for example, the following conditions can be adopted. (1) A low ionic strength and a high temperature for washing (for example, at 50°C, 0.015 M sodium chloride/0.0015 M sodium citrate/0.1% sodium dodecyl sulfate) are used, (2) a denaturant such as formamide during hybridization (for example, at 42°C, 50% (v/v) formamide and 0.1% bovine serum albumin/0.1% ficol/0.1% polyvinylpyrrolidone/50 mM sodium phosphate buffer with pH 6.5, 750 mM sodium chloride, 75 mM sodium citrate) is used, or (3) incubation is performed at 37°C overnight in a solution containing 20% formamide, 5 x SSC, 50 mM sodium phosphate (pH 7.6), 5 x Denhardt's solution, 10% dextran sulfate, and 20 mg/ml denatured sheared salmon sperm DNA, and then the filter is washed at at about 37 to 50°C and 1 x SSC. Note that a formamide concentration may be 50% or more. A washing time may be 5, 15, 30, 60, or 120 minutes or longer. A plurality of factors such as a temperature and a salt concentration are considered as factors affecting the stringency of the hybridization reaction, and for details, reference can be made to Ausubel et al., Current Protocols in Molecular Biology, Wiley Interscience Publishers, (1995). Examples of the "highly stringent conditions" are 0.0015 M sodium chloride, 0.0015 M sodium citrate, and 65 to 68°C, or 0.015 M sodium chloride, 0.0015 M sodium citrate, 50% formamide, and 42°C. The operation can be performed by hybridization and methods described in experimental textbooks such as Molecular Cloning 2nd ed., Current Protocols in Molecular Biology, Supplement 1-38, DNA Cloning 1: Core Techniques, A Practical Approach, Second Edition, Oxford University Press (1995). Here, a sequence including only the A sequence or only the T sequence is preferably excluded from a sequence that hybridizes under stringent conditions. Moderately stringent conditions can be readily determined by those skilled in the art, for example, based on DNA length, are shown in Sambrook et al., Molecular Cloning: A Laboratory Manual, No. 3, Vol. 1, 7.42-7.45 Cold Spring Harbor Laboratory Press, 2001; and for nitrocellulose filters, include use of a pre-wash solution of 5 x SSC, 0.5% SDS, 1.0 mM EDTA (pH 8.0), hybridization conditions of about 50% formamide, 2 x SSC-6x SSC (or other similar hybridization solutions such as Stark's solution in about 50% formamide at about 42°C) at about 40 to 50°C, and wash conditions of 60°C, 0.5 x SSC, and 0.1% SDS. Thus, the polypeptides used in the present disclosure also include a polypeptide encoded by a nucleic acid molecule that hybridizes under highly or moderately stringent conditions to a nucleic acid molecule encoding a polypeptide specifically described in the present disclosure.

As used herein, the "corresponding" amino acid or nucleic acid refers to an amino acid or a nucleotide which has or is expected to have, in a certain polypeptide molecule or polynucleotide molecule, a similar action as a predetermined amino acid or nucleotide in a reference polypeptide or a polynucleotide for comparison, and, particularly in the case of enzyme molecules, refers to an amino acid which is present at a similar position in an active site and makes a similar contribution to catalytic activity. For example, for an antisense molecule, it can be a similar moiety in an ortholog corresponding to a specific moiety of the antisense molecule. A corresponding amino acid can be a specific amino acid subjected to, for example, cysteination, glutathionylation, S-S bond formation, oxidation (for example, oxidation of a methionine side chain), formylation, acetylation, phosphorylation, glycosylation, myristylation, or the like. Alternatively, a corresponding amino acid can be an amino acid responsible for dimerization. Such a "corresponding" amino acid or nucleic acid may be a region or a domain over a certain range. Accordingly, it is referred as used herein as a "corresponding" region or domain in such a case. As a result of alignment between two sequences, the nucleic acids or amino acids located at the same position can be the corresponding nucleic acids or amino acids.

As used herein, the "corresponding" gene (for example, a polynucleotide sequence or molecule) refers to a gene (for example, a polynucleotide sequence or molecule) in a certain species which has or is expected to have a similar action as a predetermined gene in a reference species for comparison. When a plurality of genes having such an action are present, the corresponding gene refers to a gene having the same evolutionary origin. Accordingly, a gene corresponding to a certain gene may be an ortholog of such a gene. For example, a gene corresponding to an NRPS module for binding of a specific amino acid in a certain bacterium can be found by searching a target bacterial sequence database using a gene sequence of the NRPS module serving as a reference for the corresponding gene as a query sequence.

In accordance with the present disclosure, in the present specification, the term "activity" refers to a function of a molecule in its broadest sense. Activity, although not intended to be limiting, generally includes a biological function, biochemical function, physical function, and chemical function of a molecule. Examples of the activity include enzymatic activity, an ability to interact with another molecule, an ability to activate, promote, stabilize, inhibit, suppress, or destabilize a function of another molecule, stability, and an ability to localize at a specific position in a cell. When applicable, the term also relates to a function of a protein complex in the broadest sense.

As used herein, when referring to a certain gene or a nucleic acid molecule or a polypeptide related thereto, the "biological function" refers to a specific function that the gene, nucleic acid molecule, or polypeptide can have in vivo, and examples thereof include, but are not limited to, a specific cell surface structure recognition ability, an enzyme activity, and a binding ability to a specific protein. In the present disclosure, for example, a function in which a certain promoter is recognized in a specific host cell can be exemplified, but the present disclosure is not limited thereto. As used herein, the biological function can be exerted by "biological activity". As used herein, the "biological activity" refers to an activity that a certain agent (for example, a polynucleotide, a protein, or the like) can have in a living body. Biological activity encompasses an activity of exerting a variety of functions (for example, transcription promoting activity), and also encompasses, for example, an activity of activating or inactivating another molecule by an interaction with a certain molecule. For example, when a certain factor is an enzyme, the biological activity thereof encompasses enzyme activity thereof. In another example, in a case where a certain factor is a ligand, binding to a receptor corresponding to the ligand is encompassed. Such biological activity can be measured by a technique that is well known in the art. Thus, the "activity" refers to various measurable indicators that indicate or reveal binding (either directly or indirectly); and affect a response (that is, it has a measurable effect in response to some exposure or stimulus), and examples thereof include the amount of upstream or downstream proteins or a measure of other similar function in a host cell.

As used herein, the terms "transformation", "transduction", and "transfection" are used interchangeably, unless otherwise stated, and refer to the introduction of a nucleic acid into a host cell (optionally via a virus or a virus-derived construct). As a transformation method, any method can be used as long as it is a method of introducing a nucleic acid into a host cell, and examples thereof include various well-known techniques such as use of competent cells, an electroporation method, a method using a particle gun (gene gun), and a calcium phosphate method.

As used herein, the "purified" substance or biological factor (for example, a nucleic acid, a protein, or the like) refers to a substance or a biological factor in which at least a part of a factor naturally accompanying the biological factor is removed. Thus, the purity of the biological factor in the purified biological factor is generally higher than the purity in the normal state of the biological factor (that is, concentrated). The "purified" as used herein refers to the presence of preferably at least 75% by weight, more preferably at least 85% by weight, still more preferably at least 95% by weight, and most preferably at least 98% by weight of a biological factor of the same type. The substance used in the present disclosure is preferably a "purified" substance.

As used herein, a "drug", "agent", or "factor" (all the terms correspond to an "agent" in English) is used interchangeably in a broad sense, and may be any substance or other elements (for example, energy such as light, radioactivity, heat, and electricity) as long as the intended object can be achieved. Examples of such a substance include, but are not limited to, a protein, a polypeptide, an oligopeptide, a peptide, a polynucleotide, an oligonucleotide, a nucleotide, a nucleic acid (including, for example, DNA such as cDNA or genomic DNA, RNA such as mRNA), a polysaccharide, an oligosaccharide, a lipid, an organic small molecule (for example, a hormone, a ligand, an information transmitting substance, an organic small molecule, a molecule synthesized by combinatorial chemistry, a small molecule that can be used as a medicine (for example, a small molecule ligand or the like), or the like), a composite molecule thereof, and a mixture thereof.

As used as used herein, the "complex" or "complex molecule" means any construct containing two or more moieties. For example, when one moiety is a polypeptide, the other moiety may be a polypeptide or another substance (for example, a substrate, a sugar, a lipid, a nucleic acid, another hydrocarbon, or the like). As used herein, two or more moieties constituting the complex may be bonded by a covalent bond or may be bonded by another bond (for example, a hydrogen bond, an ionic bond, a hydrophobic interaction, van der Waals force, or the like).

As used herein, the "kit" refers to a unit generally providing portions to be provided (for example, an NRPS-based construct, instructions, and the like) that are usually divided into two or more sections. The form of the kit is preferred when it is intended to provide a composition that should not be provided in a mixed state for stability or the like, but is preferably mixed immediately before use. Such a kit preferably includes an instruction or manual describing how to use the provided portions or how a reagent should be processed. When the kit is used herein as a reagent kit, the kit generally includes an instruction or the like describing how to use a plasmid and the like.

The term "about" refers to the indicated value plus or minus 10%. The "about" when used for a temperature refers to an indicated temperature plus or minus 5°C, and the "about" when used for a pH refers to an indicated pH plus or minus 0.5.

### (Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure in view of the description as used herein. In addition, it is also understood that the following embodiments may be used alone or in combination.

### (Modified NRPS)

In one aspect, the present disclosure provides a nucleic acid containing one or a plurality of non-native restriction enzyme recognition sequences and encoding a modified NRPS. In one embodiment, the present disclosure provides a modified NRPS encoded by such a nucleic acid. The inventors have introduced a restriction enzyme recognition sequence into a nucleic acid encoding an NRPS to produce a modified NRPS, and it was found that the modified NRPS can produce a target NRP as well as the original NRPS. The introduction of the restriction enzyme recognition sequence facilitates modification of the NRPS and enables production of various NRPSs.

The modified NRPS may contain one or a plurality of NRPS modules. Each NRPS module may independently be an NRPS module derived from an organism such as the genus Bacillus (for example, B. subtilis, B. amyloliquefaciens, and B. licheniformis), Brevibacillus brevis, Paenibacillus polymyxa, Photorhabdus asymbiotica, Photorhabdus luminescens, the genus Xenorhabdus (for example, X. bovienii, X. budapestensis, X. ducetiae, X. indica, X. miraniensis, X. nematophila, X. stockiae, and X. szentirmaii), the genus Streptomyces (for example, S. verticillus and S. roseosporus), the genus Penicillium, the genus Aspergillus, the genus Tolypocladium (for example, T. inflatum and T. niveum), the genus Fusarium, the genus Microcystis, or the like. For example, one or a plurality of NRPS modules in a modified NRPS may have at least 80%, at least 85%, at least 90%, at least 95%, at least 96%, at least 97%, at least 98%, or at least 99% amino acid sequence identity to a natural NRPS module (of a particular species or strain).

Since one species or strain may contain a plurality of NRPS modules, the modified NRPS of the present disclosure may contain a plurality of NRPS modules derived from one species or strain in combination in a particular order.

In one embodiment, each domain (A domain, T domain, C domain, or the like) of the NRPS module may be replaced with a corresponding domain of the NRPS module derived from another organism (species or strain) involved in linking the same type of amino acids.

In one embodiment, in addition to the A domain, the T domain, and the C domain of the NRPS module, the modified NRPS of the present disclosure may contain other domains such as a COM domain, a TE domain, an epimerized (E) domain, a methylated (M) domain, a reduced (R) domain, a formylated (F) domain, a cyclized (Cy) domain, an oxidized (Ox) domain, a ketoacyl reductase (KR) domain, and a monooxygenase (MOx) domain (for example, see Angew Chem Int Ed Engl. 2017 Mar 27; 56 (14): 3770-3821).

In one embodiment, a modified NRPS of the present disclosure may contain a desired number of NRPS modules, for example, 3 to 100, such as 3, 4, 5, 6, 7, 8, 9, 10, 15, 20, 25, 30, 40, 50, 60, 70, 80, 90, or 100 NRPS modules.

In one embodiment, a nucleic acid encoding a modified NRPS of the present disclosure can contain a non-native restriction enzyme recognition sequence between nucleic acid sequences encoding an NRPS module derived from different organisms (species or strains). In one embodiment, a nucleic acid encoding a modified NRPS of the present disclosure can contain a non-native restriction enzyme recognition sequence between nucleic acid sequences encoding a non-native and non-contiguous NRPS module.

### (Restriction enzyme recognition sequence)

In one aspect, a nucleic acid encoding an NRPS of the present disclosure can contain one or a plurality of non-native restriction enzyme recognition sequences. In the introduction of a non-native restriction enzyme recognition sequence, base insertion, deletion, and substitution may be used in any combination. The non-native restriction enzyme recognition sequence is introduced so that no stop codon is formed and is introduced so as not to misalign a reading frame of the codon. In one embodiment, a non-native restriction enzyme recognition sequence is designed to encode a non-bulky amino acid (alanine, glycine, or the like).

In one embodiment, a non-native restriction enzyme recognition sequence is designed to be introduced into an alignment of the NRPS modules other than a consensus sequence portion with high homology. In one embodiment, when all amino acid sequences in a region from an A domain to a C domain of an NRPS module of a certain biological strain are obtained and aligned, a non-native restriction enzyme recognition sequence is introduced into a region of 3, 4, 5, 6, 7, 8, 9, or 10 amino acids in length that does not contain an amino acid position where the same amino acid is present at a proportion of more than 30%, 40%, 50%, 60%, 70%, 80%, or 90% of corresponding amino acid positions.

In one embodiment, a non-native restriction enzyme recognition sequence can be introduced into a site where a restriction enzyme recognition sequence can be introduced without changing the amino acid specified by the capitalized amino acid in the amino acid sequence of each of an A1 core motif sequence (L(TS)YxEL), an A2 core motif sequence (LKAGxAYL(VL)P(LI)D), an A3 core motif sequence (LAYxxYTSG(ST)TGxPKG), an A4 core motif sequence (FDxS), an A5 core motif sequence (NxYGPTE), an A6 core motif sequence (GELxIxGxG(VL)ARGYL), an A7 core motif sequence (Y(RK)TGDL), an A8 core motif sequence (GRxDxQVKIRGxRIELGEIE), an A9 core motif sequence (LPxYM(IV)P), an A10 core motif sequence (NGK(VL)DR), a T core motif sequence (DxFFxxLGG(HD)S(LI)), a C1 core motif sequence (SxAQxR(LM)(WY)xL), a C2 core motif sequence (RHExLRTxF), a C3 core motif sequence (MHHxISDG(WV)S), a C4 core motif sequence (YxD(FY)AVW), a C5 core motif sequence ((IV)GxFVNT(QL)(CA)xR), a C6 core motif sequence ((HN)QD(YV)PFE), and a C7 core motif sequence (RDxSRNPL), or a site that does not fall into any of these core motif sequences.

In one embodiment, the non-native restriction enzyme recognition sequence is located between base sequences encoding adjacent NRPS modules. In one embodiment, the non-native restriction enzyme recognition sequence is located between the C domain coding region and A domain coding region of the NRPS module. In one embodiment, the non-native restriction enzyme recognition sequence is located in a region between the two domain coding regions of A, T, and C of the NRPS module (for example, the C domain coding region and the A domain coding region), which does not include the two domain coding regions.

In one embodiment, the non-native restriction enzyme recognition sequence is a sequence in which a site between two restriction enzyme-specific recognition sites is cleaved, and is, for example, a recognition sequence such as SfiI (5'-GGCCNNNN/NGGCC-3'), BglI (5'-GCCNNNN/NGGC-3'), AlwNI (5'-CAGNN/CTG-3'), DraIII (5'-CACNNN/GTG-3'), PflMI (5'-CCANNNN/NTGG-3'), or BstAPI (5'-GCANNNN/NTGC-3') of TypeII restriction enzyme. In one embodiment, the non-native restriction enzyme recognition sequence is a sequence in which a site distant from a restriction enzyme-specific recognition site is cleaved, and is, for example, a recognition sequence such as AarI (5'-CACCTGCNNNN/NNNN-3'), BbsI (5'-GAAGACNN/NNNN-3'), BsaI (5'-GGTCTCN/NNNN-3'), BsmBI (5'-CGTCTCN/NNNN-3'), or BspQI (5'-GCTCTTCN/NNN-3') of TypeIIS restriction enzyme. Since the restriction enzyme-specific recognition site is different from the cleavage site, an overhang sequence of a nucleic acid fragment generated by a restriction enzyme treatment becomes variable, and therefore, a plurality of types of overhang sequences can be formed by the treatment with one type of restriction enzyme; thus, TypeII or TypeIIS restriction enzyme can be suitably used in a method using a plurality of types of overhang sequences (such as the OGAB method).

In one embodiment, a non-native restriction enzyme recognition sequence can be introduced so that a plurality of types of cleaved nucleic acid fragments do not have the same overhang sequence when a nucleic acid encoding an NRPS containing the non-native restriction enzyme recognition sequence is treated with a restriction enzyme that recognizes the restriction enzyme recognition sequence.

Any type of restriction enzyme recognition sequence may be introduced into an introduction site of any of the non-native restriction enzyme recognition sequences described above.

### (Novel NRPS production using restriction enzyme recognition sequence)

In one aspect, the present disclosure provides a method for producing a product nucleic acid containing a nucleic acid sequence encoding a non-ribosomal peptide synthetase (NRPS), the method including: a step of preparing a nucleic acid containing a non-native restriction enzyme recognition sequence as a starting nucleic acid; a step of preparing a cleaved nucleic acid fragment by treating the nucleic acid with a restriction enzyme that recognizes the restriction enzyme recognition sequence; and a step of forming a product nucleic acid that is different from the starting nucleic acid by ligating the cleaved nucleic acid fragments.

In one embodiment, in the step of ligating cleaved nucleic acid fragments, a mixture (such as a solution) that causes a ligation reaction contains a plurality of types of cleaved nucleic acid fragments, the plurality of types of cleaved nucleic acid fragments contain a plurality of types of overhang sequences, and at least some of the overhang sequences are generated by cleavage of a non-native restriction enzyme recognition sequence. When there are a pair of complementary overhang sequences among the plurality of types of overhang sequences, cleaved nucleic acid fragments containing the complementary overhang sequences can be preferentially ligated. Therefore, when there are a plurality of types of cleaved nucleic acid fragments containing the same type of overhang sequence, which cleaved nucleic acid fragment is incorporated into a position designated by the type of the overhang sequence is random, and a modified NRPS in which a specific region of an NRPS (for example, one NRPS module) is replaced can be easily obtained.

In one embodiment, the plurality of cleaved nucleic acid fragments include a set of cleaved nucleic acid fragments containing the same overhang sequence and containing a base sequence encoding a plurality of types of NRPS modules. In one embodiment, the plurality of cleaved nucleic acid fragments include a set of cleaved nucleic acid fragments containing the same overhang sequence and containing a base sequence encoding a plurality of types of NRPS modules capturing amino acids. In one embodiment, each of the cleaved nucleic acid fragments contains an overhang sequence of 3 to 5 bases in length.

Since one NRPS module can be involved in linking one amino acid in an NRPS, inclusion of an integer number of NRPS modules (equal number of A, T, and C domains) in the cleaved nucleic acid fragment may facilitate the design of NRPs produced by the produced modified NRPSs. In one embodiment, each of the cleaved nucleic acid fragments independently contains 0 to 5 (0, 1, 2, 3, 4, or 5) NRPS modules. In one embodiment, each of the cleaved nucleic acid fragments contains at most one NRPS module.

In one embodiment, in the mixture that results in the ligation reaction, the set of cleaved nucleic acid fragments containing the same overhang sequence and encoding at least a portion of the NRPS module are preferably cleaved at the corresponding site of the NRPS module. The corresponding site may be, for example, a position separated by 20 amino acids or less (for example, within 15, 10, 5, 4, 3, 2, or 1 amino acid) when the amino acid sequences of the NRPS module are aligned with each other, such as the A-T linker region of each NRPS module or a specific region of the A domain. For example, when cleaved nucleic acid fragments containing the same overhang sequence are cleaved in the A domain coding region and cleaved in the T domain coding region, continuous appearance of the A domain coding region or deletion of the A domain coding region may occur in the NRPS coding nucleic acid obtained by ligation, and thus, production of an NRP may fail.

In one embodiment, the cleaved nucleic acid fragment may contain a domain such as a COM domain, a TE domain, an epimerized (E) domain, a methylated (M) domain, a reduced (R) domain, a formylated (F) domain, a cyclized (Cy) domain, an oxidized (Ox) domain, a ketoacyl reductase (KR) domain, and a monooxygenase (MOx) domain (for example, see Angew Chem Int Ed Engl. 2017 Mar 27; 56 (14): 3770-3821) of an NRPS module. These domains can be appropriately selected by those skilled in the art according to, for example, the type of amino acid to be incorporated.

In one embodiment, when the nucleic acid encoding the NRPS module is treated with a restriction enzyme, the nucleic acid may contain a natural restriction enzyme recognition sequence by the restriction enzyme, and in this case, the overhang sequence of the nucleic acid fragment generated by treating the nucleic acid with the restriction enzyme is preferably different from any of the other nucleic acid fragments mixed with the nucleic acid fragment. When there is no other nucleic acid fragment containing the same overhang sequence, the nucleic acid fragment generated by cleavage of the natural restriction enzyme recognition sequence can return to the state before cleavage by ligation.

In one embodiment, when NRPS modules that are not naturally adjacent to each other are linked, it is necessary to link the NRPS modules in such a manner that the function of extending the peptide chain of each NRPS module is not impaired, and for example, since the C domain specifies an amino acid to be linked, it is necessary not to destroy this function.

In one embodiment, the present disclosure provides a method for producing a product plasmid containing a nucleic acid sequence encoding a non-ribosomal peptide synthetase (NRPS), the method including: a step of preparing a set of starting plasmids, each of the set of starting plasmids containing a base sequence encoding at least one NRPS module and at least one restriction enzyme recognition sequence; a step of preparing a mixture of containing a plurality of resulting cleaved nucleic acid fragments by treating the set of starting plasmids with a restriction enzyme; a step of forming a linked nucleic acid by ligating the plurality of cleaved nucleic acid fragments; and a step of bringing the linked nucleic acid into contact with a transgenic organism to form a product plasmid. In one embodiment, the mixture containing the cleaved nucleic acid fragments is a solution.

In one embodiment, each of the starting nucleic acids independently contains the same or different selectable marker sequences. In one embodiment, the selectable marker sequence includes a drug-resistant marker sequence. In one embodiment, each of the starting nucleic acids independently contains the same or different origins of replication operable in Bacillus subtilis. In one embodiment, each of the starting nucleic acids independently contains the same or different promoter regions upstream of the base sequence encoding an NRPS.

In one embodiment, the starting plasmid contains a nucleic acid sequence that promotes plasmid replication in a transgenic organism (such as Bacillus subtilis). In one embodiment, the nucleic acid sequence that promotes plasmid replication in Bacillus subtilis may contain an origin of replication that operates in Bacillus subtilis, for example, an origin of replication contained in a plasmid such as oriC, pTB19 (Imanaka,T., et al. J. Gen. Microbioi. 130, 1399-1408 (1984)) or pLS32 (Tanaka, T and Ogra, M. FEBS Lett. 422, 243-246 (1998)), or pAMβ1 (Swinfield, T. J., et al. Gene 87, 79-90 (1990)). Examples of the nucleic acid sequence that promotes plasmid replication in Bacillus subtilis include a plasmid or a portion thereof, or an origin of replication or a variant thereof, which is known to activate the replication mechanisms such as rolling circle type, theta type, oriC, and phage. In one embodiment, the nucleic acid sequence that promotes plasmid replication in Bacillus subtilis may contain a sequence that is identical or similar to a known origin of replication (for example, 90% or more, 95% or more, 97% or more, 98% or more, 99% or more, or 99.5% or more sequence identity).

In one embodiment, the starting plasmid may contain a promoter and/or enhancer that operates in Bacillus subtilis. Examples of the promoter in Bacillus subtilis include Pspac (Yansura, D. and Henner, D. J. Pro. Natl. Acad. Sci, USA 81, 439-443 (1984)) whose expression can be controlled by isopropyl s-D-thiogalactopyranoside (IPTG), and a Pr promoter (Itaya, M. Biosci. Biotechnol. Biochem. 63, 602-604 (1999)). The nucleic acid element that operates in Bacillus subtilis does not need to be derived from Bacillus subtilis, and a nucleic acid element that operates with high efficiency or the like can be selected.

In one embodiment, the present disclosure provides a method for producing an NRPS-derived construct plasmid of the present disclosure, the method including a step of operably linking a plurality of nucleic acid sequences. In one embodiment, since Bacillus subtilis may have an ability to form a plasmid from a nucleic acid taken in from the outside, in the method, the nucleic acid to be introduced may not be a plasmid. For example, when being in contact with a nucleic acid (for example, an acyclic nucleic acid containing a tandem repeat nucleic acid sequence), Bacillus subtilis can take up the nucleic acid and form a plasmid within Bacillus subtilis (see, for example, the OGAB method described in the present specification).

In one embodiment, a method for introducing a nucleic acid into Bacillus subtilis may be any method, and examples thereof include use of competent Bacillus subtilis, an electroporation method, a method using a particle gun (gene gun), and a calcium phosphate method. The "competent" refers to a state in which a cell becomes more permeable to an exogenous substance (for example, a nucleic acid). Any known method can be used to render Bacillus subtilis competent, but for example, the methods described in Anagnostopoulou, C. and Spizizen, J. J. Bacteriol., 81, 741-746 (1961) can be used. In one embodiment, the NRPS-derived construct plasmid of the present disclosure may be produced in Bacillus subtilis and directly amplified in the same Bacillus subtilis.

### (OGAB method)

In one embodiment, a plasmid or plasmid library encoding an NRPS of the present disclosure can be produced by the OGAB method. The ordered gene assembly in Bacillus subtilis (OGAB) method is a method for producing a circular plasmid in a transgenic organism by incorporating an assembled nucleic acid into the organism. In the OGAB method, a plasmid containing a large sized nucleic acid can be conveniently prepared. The nucleic acid to be incorporated into the transgenic organism is referred to as "assembled nucleic acid" in the present specification, typically, the assembled nucleic acid has a tandem repeat-like structure in which one unit of the plasmid and one set of unit nucleic acids repeatedly appear in the same direction, and the use of the assembled nucleic acid having such a structure can promote the production of the plasmid in the transgenic organism. As used herein, the "unit nucleic acid" refers to a nucleic acid molecule or a portion of a nucleic acid molecule having a part of a sequence constituting the sequence of the assembled nucleic acid, and as described in detail below, a plurality of types of unit nucleic acids are prepared as a unit vector, and then the assembled nucleic acid is constructed.

Hereinafter, a procedure for producing an assembled nucleic acid to be incorporated into a transgenic organism will be described.

### • Preparation of unit nucleic acid

A unit nucleic acid for incorporation into an assembled nucleic acid is prepared. The unit nucleic acid can be produced by any known method, for example, by polymerase chain reaction (PCR) or chemical synthesis. The unit nucleic acid may contain any desired sequence such as a sequence encoding a desired protein (such as an NRPS) or a portion thereof, a sequence controlling a gene (a promoter, an enhancer, or the like), or a sequence for manipulating a nucleic acid (such as a restriction enzyme recognition sequence or the like). An end of each unit nucleic acid can be configured to produce a particular overhang sequence so that, when incorporated into an assembled nucleic acid, a plurality of types of unit nucleic acids are aligned in a particular order and/or orientation of property.

Since a large number of unit nucleic acids can ultimately be assembled onto a plasmid, one or a plurality of unit nucleic acids may be designed to encode one or a plurality of genes each having a long base length. Examples of the gene having a long base length include a group of genes constituting an NRPS complex.

### • Preparation of unit vector

A unit vector can be prepared by linking a unit nucleic acid and an additional nucleic acid different from the unit nucleic acid. The use of the unit vector may allow for easier handling of the unit nucleic acid.

The additional nucleic acid may be a linear nucleic acid or a circular plasmid. When a circular plasmid is used as an additional nucleic acid, a unit vector can also have a circular structure, and thus can be used for transformation of, for example, E. coli or the like. In one embodiment, the additional nucleic acid can contain an origin of replication so that the unit vector is replicated in a target host. In one embodiment, all the unit nucleic acids for constructing a certain assembled nucleic acid may be linked to the same type of additional nucleic acid, thereby reducing a size difference between the unit vectors and facilitating the handling of a plurality of types of unit vectors. In one embodiment, a unit nucleic acid for constructing an assembled nucleic acid may be linked to different types of additional nucleic acids. In one embodiment, for one or a plurality of unit nucleic acids for constructing an assembled nucleic acid, a ratio of (base length of unit nucleic acid)/(base length of unit vector) or an average thereof can be 50% or less, 40% or less, 30% or less, 20% or less, 15% or less, 10% or less, 7% or less, 5% or less, 2% or less, 1.5% or less, 1% or less, or 0.5% or less. As the size of the additional nucleic acid is larger than the unit nucleic acid, the handling of different types of unit vectors can be more uniform. The linking between the unit nucleic acid and the additional nucleic acid can be performed by any method such as ligation using DNA ligase or TA cloning. In one embodiment, for one or a plurality of unit nucleic acids for constructing an assembled nucleic acid, a ratio of (base length of unit nucleic acid)/(base length of unit vector) or an average thereof can be 1% or more, 0.3% or more, 0.1% or more, 0.03% or more, 0.01% or more, 0.003% or more, or 0.001% or more, and the manipulation of the unit vector may be facilitated.

In one embodiment, the length of the unit nucleic acid may be 10 bp or more, 20 bp or more, 50 bp or more, 70 bp or more, 100 bp or more, 200 bp or more, 500 bp or more, 700 bp or more, 1,000 bp or more, or 1,500 bp or more and 5,000 bp or less, 5,000 bp or less, 2,000 bp or less, 1,500 bp or less, 1,200 bp or less, 1,000 bp or less, 700 bp or less, or 500 bp or less.

In one embodiment, the assembled nucleic acid can be constructed from 2 or more, 4 or more, 6 or more, 8 or more, 10 or more, 15 or more, 20 or more, 30 or more, 40 or more, 50 or more, 60 or more, 70 or more, 80 or more, 90 or more, or 100 or more, and 1,000 or less, 700 or less, 500 or less, 200 or less, 120 or less, 100 or less, 80 or less, 70 or less, 60 or less, 70 or less, or 50 or less unit nucleic acids. By adjusting the number of moles of each unit nucleic acid (or unit vector) to be substantially the same, a desired assembled nucleic acid having a tandem repeat-like structure can be efficiently produced.

In one embodiment, the unit nucleic acid may have a base length obtained by approximately equally dividing one set of repeat sequences in the assembled nucleic acid by the number of unit nucleic acids. This may facilitate a manipulation of aligning the number of moles of each unit nucleic acid (or unit vector). In one embodiment, the unit nucleic acid may have an increased or decreased base length of 30% or less, 25% or less, 20% or less, 15% or less, 10% or less, 7% or less, or 5% or less from a base length obtained by approximately equally dividing one set of repeat sequences in the assembled nucleic acid by the number of unit nucleic acids.

In one embodiment, the unit nucleic acid can be designed to have a non-palindromic sequence (a sequence that is not a palindromic sequence) at the end of the unit nucleic acid. The unit nucleic acid designed in this way can easily provide a structure in which the unit nucleic acids are linked to each other while maintaining the order in the assembled nucleic acid when the non-palindromic sequence is made into an overhang sequence.

### • Production of assembled nucleic acid

The assembled nucleic acid can be constructed by linking the unit nucleic acids to each other. In one embodiment, the unit nucleic acid can be prepared by being cut out from the unit vector by a restriction enzyme or the like. An assembled nucleic acid may contain one or more, two or more, three or more, four or more, five or more, six or more, seven or more, eight or more, nine or more, or ten or more sets of repeat sequences. The repeat sequence in the assembled nucleic acid may include a sequence of a unit nucleic acid and, as necessary, a sequence of an assembled vector nucleic acid. The assembled nucleic acid may contain a sequence that enables replication of a nucleic acid in a transgenic organism. In one embodiment, a sequence that allows replication of a nucleic acid in a transgenic organism can contain an effective origin of replication in a transgenic organism (for example, the genus Bacillus (Bacillus subtilis)). The sequence of the effective origin of replication in Bacillus subtilis is not particularly limited, and examples of a sequence having a θ-type replication mechanism include a sequence of an origin of replication contained in a plasmid such as pTB19 (Imanaka,T., et al. J. Gen. Microbioi. 130, 1399-1408 (1984)) or pLS32 (Tanaka, T and Ogra, M. FEBS Lett. 422, 243-246 (1998)), or pAMβ1 (Swinfield, T. J., et al. Gene 87, 79-90 (1990)).

The assembled nucleic acid may contain an additional base sequence, as necessary, in addition to the unit nucleic acid. In one embodiment, the assembled nucleic acid may contain a base sequence that controls transcription translation, such as a promoter, an operator, an activator, or a terminator. Specific examples of a promoter in a case of using Bacillus subtilis as a host include Pspac (Yansura, D. and Henner, D. J. Pro. Natl. Acad. Sci, USA 81, 439-443 (1984)) whose expression can be controlled by isopropyl s-D-thiogalactopyranoside (IPTG), and a Pr promoter (Itaya, M. Biosci. Biotechnol. Biochem. 63, 602-604 (1999)).

The unit nucleic acid may form a repeating structure that maintains a certain order and orientation in the assembled nucleic acid. In one embodiment, the unit nucleic acid is constructed so that the base sequences at the overhang ends of the unit nucleic acid cut out from the unit vector are complementary to each other, such that it is possible to form a repeating structure in which a certain order and orientation in the assembled nucleic acid are maintained. In one embodiment, the structure of the overhang end is made unique for each different unit nucleic acid, such that it is possible to efficiently form a repeat structure maintaining a certain order and orientation. In one embodiment, the overhang ends may have a non-batch sequence and may be either a 5'-terminal overhang or a 3'-terminal overhang.

In one embodiment, a unit nucleic acid having an overhang end can be cut out from a unit vector using a restriction enzyme. In the embodiment, the unit vector may contain one or a plurality of restriction enzyme recognition sequences. In a case where the unit vector contains a plurality of restriction enzyme recognition sequences, each restriction enzyme recognition sequence may be recognized by the same restriction enzyme or may be recognized by different restriction enzymes. In one embodiment, the unit vector can contain a pair of regions recognized by the same restriction enzyme so that a complete unit nucleic acid region is included between these regions. The restriction enzyme used are not particularly limited, examples thereof include type II restriction enzymes, for example, AarI, BbsI, BbvI, BcoDI, BfuAI, BsaI, BsaXI, BsmAI, BsmBI, BsmFI, BspMI, BspQI, BtgZI, FokI, SfaNI, and the like, and these restriction enzymes can create an overhang end at a site at a certain distance from the recognition sequence. Using a (for example, single) type IIS restriction enzyme can be advantageous for assembling a plurality of unit nucleic acids in a certain order and orientation, because the sequence of the overhang end of the cut unit nucleic acid may be different for each unit nucleic acid. In one embodiment using the type IIS restriction enzyme, the unit vector does not contain a region recognized by the type IIS restriction enzyme in the unit nucleic acid region. In an embodiment using a restriction enzyme that cleaves a recognition region, the unit vector may contain a region recognized by the restriction enzyme at the end of the unit nucleic acid region.

In one embodiment, when the same type of restriction enzyme is used to cut out the unit nucleic acid from a plurality of unit vectors, the restriction enzyme treatment can be performed in a solution containing the plurality of unit vectors, and the efficiency of the operation can be improved. The types of restriction enzymes used to produce a certain assembled nucleic acid may be, for example, 5 or less, 4 or less, 3 or less, 2 or less, or 1, and the use of a small number of restriction enzymes can reduce a variation in the number of moles between unit nucleic acids. In one embodiment, the unit nucleic acid cut out from the unit vector can be readily purified by any known fractionation method such as agarose gel electrophoresis.

The unit nucleic acids and, as necessary, the assembled vector nucleic acids can be linked (ligated) to each other using DNA ligase or the like. Therefore, an assembled nucleic acid can be produced. For example, linking of unit nucleic acids, and as necessary, assembled vector nucleic acids can be performed in the presence of components such as polyethylene glycol (for example, PEG2000, PEG4000, PEG6000, PEG8000, or the like) and a salt (for example, monovalent alkali metal, sodium chloride, or the like). A concentration of each unit nucleic acid in a ligation reaction solution is not particularly limited, and may be 1 fmol/µl or more. A reaction temperature and time of the ligation are not particularly limited, and are, for example, 30 minutes or longer at 37°C. In one embodiment, prior to ligating the unit nucleic acid and, as necessary, the assembled vector nucleic acid, the composition containing the unit nucleic acid and, as necessary, the assembled vector nucleic acid may be subjected to any conditions that inactivate a restriction enzyme (for example, phenol and chloroform treatment).

The unit nucleic acid can be adjusted to about the same number of moles using a method described in WO 2015/111248 A or the like. By adjusting the unit nucleic acid to substantially the same number of moles, a desired assembled nucleic acid having a tandem repeat-like structure can be efficiently produced. The number of moles of the unit nucleic acid can be adjusted by measuring the concentration of the unit vector or the unit nucleic acid.

### • Production of plasmid using assembled nucleic acid

Bringing the assembled nucleic acid into contact with a transgenic organism can form a plasmid in the transgenic organism. In one embodiment, examples of the transgenic organism include the genus Bacillus, the genus Streptococcus, the genus Haemophilus, and the genus Neisseria. Examples of the genus Bacillus include B. subtilis (Bacillus subtilis), B. megaterium (Bacillus megaterium), and B. stearothermophilus (Bacillus stearothermophilus). In a preferred embodiment, the transgenic organism is Bacillus subtilis. In one embodiment, the transgenic organism that incorporates the assembled nucleic acid is in a competent state and is able to actively incorporate the nucleic acid. For example, Bacillus subtilis in a competent state cleaves a double-stranded nucleic acid as a substrate on a cell, decomposes any one strand of the double-stranded nucleic acid from the cleavage point, and incorporates the other single strand into a bacterial cell. The incorporated single strand can be repaired into a circular double-stranded nucleic acid in the bacterial cell. While any known method can be used to render a transgenic organism competent, for example, Bacillus subtilis can be rendered competent using the methods described in Anagnostopoulou, C. and Spizizen, J. J. Bacteriol., 81, 741-746 (1961). As a method of transformation, a known method suitable for each transgenic organism can be used.

A plasmid produced using transformed cells can be purified using any known method, and the present disclosure also provides a plasmid thus purified. In one embodiment, it can be confirmed that the purified plasmid contains a desired nucleic acid sequence by examining the size pattern of fragments generated by restriction enzyme cleavage, a PCR method, a base sequencing method, or the like. In one embodiment, there is provided Bacillus subtilis containing a plasmid of the present disclosure.

In one embodiment, the present disclosure provides a plasmid library encoding various NRPSs produced by the method of the present disclosure. In one embodiment, the present disclosure provides a library of Bacillus subtilis containing a plasmid encoding various NRPSs produced by the method of the present disclosure.

In one embodiment, the present disclosure provides an NRPS produced using a plasmid encoding an NRPS produced by the method of the present disclosure. In one embodiment, the present disclosure provides an NRPS library produced using a plasmid encoding various NRPSs produced by the method of the present disclosure.

### (Composition)

A peptide produced using an NRPS (NRP) as described as used herein or a composition containing the same can be used in a variety of applications, such as therapy, public health, and biotechnology depending on the peptide. In one embodiment, the present disclosure provides a composition containing a peptide produced using an NRPS (NRP) as described as used herein. The composition may be provided in a variety of forms. The form of the composition may be, for example, an injection, a capsule, a tablet, a granule, an inhalant, or the like. An aqueous solution for injection may be stored, for example, in a vial or in a stainless container. In addition, the aqueous solution for injection may contain, for example, physiological saline, sugar (for example, trehalose), NaCl, NaOH, or the like.

In one embodiment, the composition of the present disclosure contains a pharmaceutically acceptable carrier or excipient. Such a carrier can be an aseptic liquid, for example, water or oil, includes a carrier derived from petroleum, an animal, a plant, or a synthetic origin, and includes, but is not limited to, peanut oil, soybean oil, mineral oil, sesame oil, and the like. Examples of the excipient include light anhydrous silicic acid, crystalline cellulose, mannitol, starch, glucose, lactose, sucrose, gelatin, malt, rice, wheat flour, chalk, silica gel, sodium stearate, glycerol monostearate, talc, sodium chloride, powdered skim milk, glycerol, propylene, glycol, water, ethanol, carmellose calcium, carmellose sodium, hydroxypropyl cellulose, hydroxypropyl methylcellulose, polyvinyl acetal diethylamino acetate, polyvinylpyrrolidone, gelatin, medium-chain fatty acid triglyceride, polyoxyethylene hydrogenated castor oil 60, poloxamer, saccharose, carboxymethylcellulose, corn starch, and an inorganic salt. The composition can contain a small amount of a wetting agent or an emulsifier, or a pH buffer, if desired. These compositions can be in the form of a solution, suspension, emulsion, tablet, pill, capsule, powder, sustained release mixture, or the like. The composition can also be formulated as a suppository using a traditional binding agent and carrier, for example, triglyceride. Oral formulation can also contain a standard carrier such as medicine grade mannitol, lactose, starch, magnesium stearate, sodium saccharin, cellulose, or magnesium carbonate. Examples of a preferred carrier are described in E. W. Martin, Remington's Pharmaceutical Sciences (Mark Publishing Company, Easton, U.S.A). In addition, for example, a surfactant, an excipient, a coloring agent, a flavoring agent, a preservative, a stabilizer, a buffer, a suspension, an isotonizing agent, a binder, a disintegrant, a lubricant, a fluidity accelerator, a corrigent, or the like may be contained. Any component of the composition of the present disclosure can be provided as a pharmaceutically acceptable salt.

As used herein, "or" is used when "at least one or more" of the items listed in the text can be employed. The same applies to "or". When explicitly described in the present specification as "within the range" of "two values", the range also includes the two values themselves.

Reference literatures such as scientific literatures, patents, and patent applications cited in the present specification are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples that are specifically described in the present specification, but is limited only by the claims.

### Examples

For reagents, the specific products described in the Examples were used. However, the reagent can be substituted with an equivalent product from another manufacturer (Sigma-Aldrich, Wako Pure Chemical, Nacalai Tesque, R&D Systems, USCN Life Science INC, or the like).

### Strain

DH5a, TOP10, and JM109 were used as E. coli for gene cloning. As a host for genetic recombination of Bacillus subtilis, 168 strain-derived strains were used. Specifically, when plipastatin NRPSs were assembled by the OGAB method, BEST9731 strain deficient in ppsABCDE, srfAA, AB, and AC was used. As a recombinant NRPS expressing host, BUSY9621 strain in which ppsABCDE was deleted and 168 sfp gene deletion was complemented was used. BEST8628 strain producing plipastatin was used for cloning of a wild-type ppsABCDE gene by the BReT method.

### Medium

LB medium was prepared by dissolving 10 g of Bactotryptone, 5 g of yeast extract, and 5 g of sodium chloride in 1 L of water, further adding 15 g of Bacto Agar in the case of an agar plate, and performing autoclaving (121°C, 20 minutes). As necessary, carbenicillin (final concentration: 100 µg/mL) or tetracycline (final concentration 10 µg/mL) was added. TF-I medium and TF-II medium for Bacillus subtilis transformation were prepared as follows. First, 10 × Spizizen (140 g of K₂HPO₄ (anhydrous), 60 g of KH₂PO₄ (anhydrous), 20 g of (NH₄)₂SO₄, and 10 g of Na₃-citric acid·₂H₂O, per 1 L), 50% glucose, 2% MgSO₄·7H₂O, 2% casamino acid, and water were individually prepared by autoclaving. In addition, an aqueous solution (5 mg/mL) of each amino acid of tryptophan, arginine, leucine, and threonine was prepared by filter sterilization. TF-I medium (500 mL) was prepared by mixing 50 mL of 10 × Spizizen, 5 mL of each of 50% glucose, 2% MgSO₄·7H₂O, 2% casamino acid, and each of 5 mg/mL amino acids of tryptophan, arginine, leucine, and threonine, and finally 415 mL of sterilized water, and performing filter sterilization, and stored at 4°C until use. TF-II medium (500 mL) was prepared by filter sterilization using the same medium as TF-I medium except that 2.5 mL of 2% casamino acid, 0.5 mL of each amino acid solution (5 mg/mL), and 435.5 mL of sterile water were used, and stored at 4°C until use. ACS medium for peptide production by Bacillus subtilis was prepared as follows. 100 g of sucrose, 11.7 g of citric acid, 4 g of sodium sulfate, 5 g of yeast extract, 4.2 g of diammonium hydrogen phosphate, 0.76 g of potassium chloride, 0.42 g of magnesium chloride hexahydrate, 0.0104 g of zinc chloride, 0.0245 g of ferric chloride hexahydrate, and 0.0181 g of manganese chloride tetrahydrate were dissolved per 1 L of a medium, and the solution was adjusted to pH 6.9 with aqueous ammonia and then autoclaved.

### In vitro genetic manipulation

Other common DNA manipulations were performed according to the standard protocol (Sambrook, J., et al., Molecular Cloning: A Laboratory Manual.Cold Spring Harbor Laboratory Press, Cold Spring Harbor, New York (1989)).

### Bacillus subtilis transformation method

2 mL of LB medium was placed in a 14 mL test tube (2051) of Falcon, and Bacillus subtilis in a glycerol stock stored at -70°C was inoculated therein and cultured at 37°C for 17 hours while being rotated by a rotary culture apparatus. Into a new 14 mL test tube (Falcon 2051), 900 µL of TF-I medium was dispensed, 25 µL of 2% casamino acid was added, 50 µL of a pre-culture solution was added, and culture was performed at 37°C for 4 hours while being rotated by the rotary culture apparatus. Thereafter, into a new 14 mL test tube, 900 µL of TF-II medium was dispensed, 100 µL of a TF-I culture solution was added, and culture was performed at 37°C for 1.5 hours while being rotated by the rotary culture apparatus. 100 µL of the TF-II culture solution was placed in a 1.5 mL centrifuge tube, and 8 µL of DNA was added thereto. Culture was performed at 37°C for 30 minutes while being rotated by the rotary culture apparatus, 300 µL of LB medium was added, and culture was further performed at 37°C for 1 hour while being rotated by the rotary culture apparatus. Thereafter, the resulting product was spread on an LB medium agar plate containing 10 µg/mL tetracycline and incubated at 37°C overnight to obtain a transformant.

### Amplification of unit gene by PCR method and cloning into plasmid

2.5 µL of 10 × an enzyme for TAKARA Ex-Taq, 2 µL of a 2.5 mM dNTP solution, and 10 pmol/µL of a primer set were each added in an amount of 0.25 µL, 1 µL of genomic DNA of Bacillus subtilis as a template, 16 µL of sterile water, and 0.5 µL of Ex-TaqHS were added, and the mixture was incubated at 94°C for 5 minutes, and then subjected to 30 cycles of 20 seconds at 98°C, 30 seconds at 58°C, 72°C, and 1 min/kb, thereby amplifying DNA. For the resulting DNA fragment, pCR-XL-TOPO Kit (Invitrogen) was used according to the manual. Alternatively, when cloning was performed by the TA cloning method using a TA cloning vector produced by cutting the plasmid pBR-delTypeIIS-XcmI with XcmI, the PCR product was purified using MiniElute PCR purification Kit, 1 µL of the PCR product was mixed with 1 µL of 10 ng/µL of the vector fragment, 2 µL of TAKATA Ligation (Mighty) Mix was added thereto, and then, the mixture was incubated at 16°C overnight. 4 µL of the ligation solution was added to 40 µL of E. coli DH5α or a chemical competent cell of JM109, the mixture was kept at a constant temperature on ice for 15 minutes, subjected to a heat shock at 42°C for 30 seconds, and left on ice for 2 minutes, 200 µL of LB medium was added, and then, the mixture was kept at a constant temperature at 37°C for 1 hour, smeared on an LB plate containing 1.5% agar containing carbenicillin at a concentration of 100 µg/mL, and cultured at 37°C overnight, thereby obtaining a plasmid transformant.

### E. coli plasmid purification and restriction enzyme cleavage

Each of the E. coli transformants with plasmids cloning DNA fragments containing desired sequences was cultured at 37°C overnight and 120 spm in 2 mL of LB medium containing 100 µg/mL carbenicillin, and the plasmids were purified on the resulting bacterial cells using QIA spin miniprep kit (Qiagen) according to the manual. 50 µL of a 10 × Cut smart buffer and 5 µL of SfiI restriction enzyme (NEB) were added to 40 µL of a DNA solution containing about 20 µg of the plasmid, and the mixture was reacted at 50°C for 2 hours to separate the unit DNA fragment from the plasmid vector.

### Size fractionation of DNA by low-melting-point agarose gel

A voltage of 50 V (about 4 V/cm) was applied to a restriction enzyme-treated DNA solution in a 1% low-melting-point agarose gel (2-Hydroxyethyl Agarose TypeVII, Sigma) in the presence of 1 × TAE buffer (Tris-Acetate-EDTA Buffer) using a general-purpose agarose gel electrophoresis apparatus (i-MyRun. N nucleic acid electrophoresis system, Cosmo Bio Co., Ltd.), and the plasmid vector and the DNA fragment were separated by electrophoresis for 1 hour. The electrophoresis gel was stained with 100 mL of 1 × TAE buffer containing 1 µg/mL ethidium bromide (Sigma) for 30 minutes, and visualized by being irradiated with ultraviolet rays having a long wavelength (366 mn), such that a DNA fragment having a predetermined size was cut out with a razor and recovered in a 1.5 mL tube. To the recovered low-melting-point agarose gel (about 300 mg), 1 × TAE buffer was added to obtain a total volume of about 700 µL, and the resulting product was kept at 65°C for 10 minutes to dissolve the gel. Thereafter, an equal amount of TE saturated phenol (Nacalai Tesque Inc.) was added and mixed well to deactivate the restriction enzyme. A phenol phase and an aqueous phase were separated by centrifugation (20,000 × g, 10 minutes), and the aqueous phase (about 900 µL) was recovered in a new 1.5 mL tube. 500 µL of 1-butanol (Wako Pure Chemical Industries, Ltd.) was added thereto, the mixture was well mixed, separation was performed by centrifugation (20,000 × g, 1 minute), and an operation of removing 1-butanol saturated with water was repeated until the volume of the aqueous phase became 450 µL or less, thereby reducing the volume of the aqueous phase. 50 µL of a 3 M potassium acetate-acetic acid buffer (pH 5.2) and 900 µL of ethanol were added thereto, the mixture was centrifuged (20,000 × g, 10 minutes) to precipitate DNA, and the resulting product was rinsed with 70% ethanol and dissolved in 20 µL of TE.

### DNA concentration adjustment

To 25 µL of DNA to be measured whose concentration was adjusted to about several ng/µL, 125 µL of a working solution obtained by diluting SYBRgreenI (Molecular Probe) with TE to 1/12,500 was added, and the mixture was well mixed to obtain a measurement sample. A known concentration of DNA (TOYOBO λ/HindIII DNA (250 µg/mL)) was diluted with TE to prepare 12 dilution series of 2 times from 31.25 ng/µL, and 125 µL of the working solution was similarly added to 25 µL of the resulting product and mixed well to obtain a standard dilution series. The measurement sample and the dilution series standard were transferred to a black 96-well plate, the fluorescence intensity was measured at an excitation wavelength of 485 nm and a fluorescence wavelength of 535 nm with a fluorescence plate reader (Thermofisher Fluoroskan FL), and the DNA concentration of the measurement target was determined by a calibration curve created from the standard dilution series.

### Gene assembly by OGAB method

The vector for gene assembly and each DNA fragment to be assembled were mixed so as to be contained in an equimolar amount of 10 fmol, and the total volume was adjusted with TE to 10 µL. 11 µL of a 2 × ligation buffer (20% (w/v) PEG6000, 132 mM Tris-HCl (pH 7.6), 13.2 mM MgCl₂, 20 mM DTT, 0.2 mM ATP, 300 mM NaCl) was added thereto, the whole mixture was kept at 37°C for 5 minutes, 1 µL of T4DNA ligase (Takara) was added, and then, the mixture was kept at 37°C for 4 hours. After confirming that 10 µL of the resulting product was ligated by electrophoresis, the resulting product was recovered in 10 µL of a new tube, 100 µL of Bacillus subtilis competent cells were added thereto, and rotary culture was performed at 37°C for 30 minutes with a duck rotor. Thereafter, 300 µL of LB medium was added, rotary culture was performed at 37°C for 1 hour with a duck rotor, and thereafter, the culture solution was spread on an LB plate containing 10 µg/mL tetracycline, and culture was performed at 37°C overnight.

### Mass preparation of plasmid by ultracentrifugation

After completion of the culture, 50 mL of the resulting product was dispensed into four 50 mL tubes (Falcon 2070) and centrifuged at 5,000 × g for 10 minutes. The supernatant was discarded, and the bacterial pellets were completely loosened by vortexing. In the case of E. coli, P1 Buffer (QIAGEN N.V.) was used alone, and in the case of Bacillus subtilis, a P1 Buffer (QIAGEN N.V) solution to which 10 mg/mL lysozyme and 10 mg/mL ribonuclease A were added was used, and 5 mL of the solution was added to each of the four tubes containing the bacteria and mixed well. The resulting product was incubated at room temperature for 5 minutes. To each of the four tubes, 5 mL of P2 Buffer (QIAGEN N.V.) was added, mixed slowly, and incubated at room temperature for 5 minutes. Furthermore, 5 mL of each P3 Buffer (QIAGEN N.V) was added, and mixing was performed with a strong force to some extent so that the cloudy substance was uniformly dispersed. Centrifugation was performed at 5,000 x g for 10 minutes, and the supernatant was pipetted and transferred to four new 50 mL tubes with a screw cap (Falcon 2070). To each tube, 5 mL of phenol saturated TE was added and mixed vigorously. Centrifugation was performed at 5,000 x g for 10 minutes, and the supernatant was pipetted and transferred to four new 50 mL tubes with a screw cap (Falcon 2070). 20 mL of each 100% ethanol was added, mixed, and centrifuged at 5,000 × g for 10 minutes, and the supernatant was removed. 5.4 mL of TE was added to the precipitate and completely dissolved. Next, 6.40 g of cesium chloride was charged and completely dissolved. Further, 2.6 mL of a 1.1 g/mL cesium chloride solution (solution prepared by mixing 1.1 g of cesium chloride and 1 mL of water, without volume adjustment) was added. Finally, 600 µL of a 10 mg/mL ethidium bromide solution was added and mixed well. The contents were transferred to one ultracentrifuge tube (Beckman 362181). Water or a 1.1 g/mL cesium chloride solution (specific gravity: about 1.5 g/mL) was added to finely adjust the weight so that the difference from the balance was within 20 mg. Centrifugation was performed using an ultracentrifuge (Beckman Coulter, Inc.) under the following conditions. Temperature: 18°C, speed: 50,000 rpm, acceleration: Max, deceleration: Max. Centrifugation was performed for 15 hours or longer.

After completion of the centrifugation, a 1 mL syringe with a needle (21G × 5/8") set was inserted into a band of a ccc type plasmid under ultraviolet (365 nm) observation, and the plasmid solution was recovered and transferred to a 15 mL tube. 500 µL of P3 was added thereto, and then, water was added so that the total volume was 3 mL. In addition, 9 mL of 100% ethanol was added. Centrifugation was performed at 5,000 × g for 10 minutes, and the supernatant was removed. 700 µL of TE was added to the resulting precipitate to dissolve the DNA. The resulting product was transferred to a 1.5 mL tube, 600 µL of 1-butanol was added and mixed, centrifuged at 20,000 × g for about 10 seconds, and separated into two layers, and the upper butanol layer was discarded. 600 µL of 1-butanol was newly added and mixed, centrifuged at 20,000 × g for about 10 seconds, and separated into two layers, and the upper butanol layer was discarded. The operation was continued until the aqueous layer became 450 µL or less. When the aqueous layer became 450 µL or less, 50 µL of P3 Buffer (QIAGEN N.V.) was added, 900 µL of ethanol was further added and mixed, and then, the mixture was centrifuged at 20,000 × g for 10 minutes. The supernatant was removed, and the resulting pellet DNA was rinsed with 900 µL of 70% ethanol and then dissolved in 22 µL of TE.

### Analysis of lipopeptide by analytical HPLC

The culture solution was placed in a 50 mL centrifuge tube, 12 N hydrochloric acid was added thereto to adjust the pH to 2, and the generated precipitate was recovered as a pellet by centrifugation at 9,160 × g for 10 minutes. 4 mL of 95% ethanol was added thereto, mixing was performed with a magnetic stirrer for 1 hour, and then, centrifugation was performed at 9,160 × g for 10 minutes to recover a supernatant. The resulting product was passed through a 0.45 µm PTFE filter to prepare an HPLC sample. HPLC was performed on a column with Inertsil ODS-2 ([4.6 mm in diameter x 250 mm in length]; GL Sciences Inc.). As the eluent, two types of organic solvents of acetonitrile:isopropyl alcohol = 7 : 3, containing (A) a 0.05% trifluoroacetic acid aqueous solution and (B) 0.02% trifluoroacetic acid, were used. The two eluents were always flowed so that the total of the two eluents was 1 mL/min. A temporal change of each eluent is as follows. At the start (0 minutes), A was linearly decreased and B was changed to increase at A = 60% and B = 40%, after 30 minutes, A = 0% and B = 100% were obtained, the same sate was maintained for 5 minutes, and then, A = 60% and B = 40% were maintained for 10 minutes. Detection was performed at absorption of 205 nm.

### Peptide purification by mass preparative HPLC

A 500 mL conical flask was charged with 200 mL of an ACS medium to which tetracycline (10 µg/mL) was added, and the bacteria were inoculated and cultured at 30°C and 120 spm for 4 days. The culture solution was adjusted to pH 2 with 12 N hydrochloric acid, and the generated precipitate was recovered by centrifugation at 9,160 × g for 10 minutes. 500 mL of 95% ethanol was added, and the mixture was eluted by stirring and then filtered by passing through a 0.45 µm filter (Corning (registered trademark) 150 mL Filter System, 0.45 µm 13.6 cm² Cellulose Acetate). 300 mL of the resulting filtrate was concentrated to 100 mL by a rotary evaporator. Again, the mixture was filtered through a 0.45 µm filter and concentrated to 10 to 15 mL by the rotary evaporator. Further, the mixture was concentrated by a vacuum centrifugal concentrator (Labconco Centrivap concentrator), and then passed through a 0.45 µm PTFE filter (htslabs.com, Standard/Filtervial PTFE 0.45 µM W/Pre-slit septum Blue snap cap PK100) to obtain a sample for mass preparative HPLC. An HPLC apparatus manufactured by Shimadzu Corporation including a fraction collector (FRC-10 A) was equipped with Phenomenex, Luna (registered trademark) 5 µm C18 (2) 100Å, LC Column 250 x 10 mm, Ea on a column, and conditions of a column temperature of 40°C and a flow rate of 3 mL/min were used. As the eluent, A and B that were the same as those of the HPLC for analysis were used, and the same conditions as those of the HPLC for analysis were used for the time change of the ratio of A and B. For detection, 205 nm was used.

### Example 1: Introduction of SfiI site into plipastatin NRPS

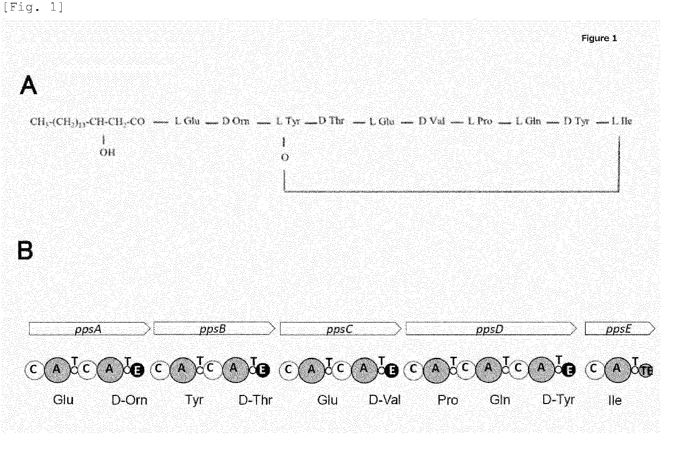

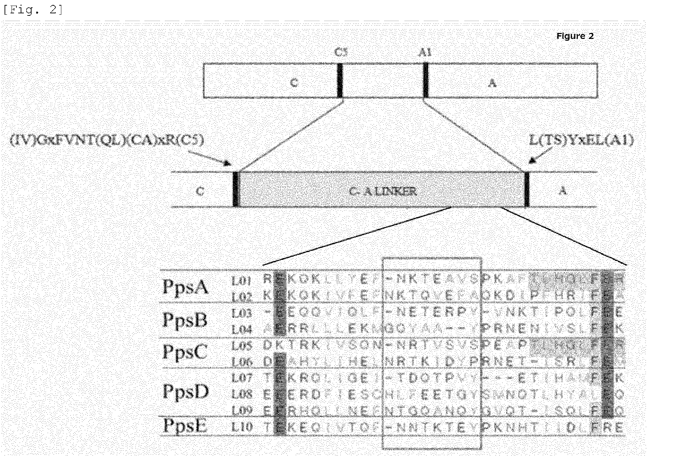

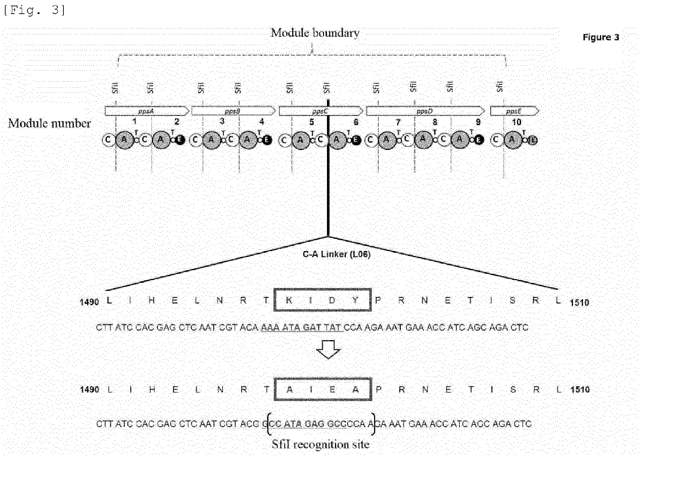

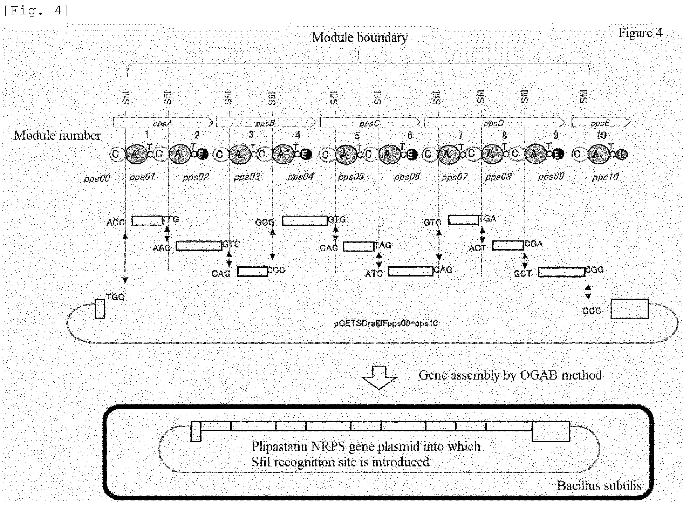

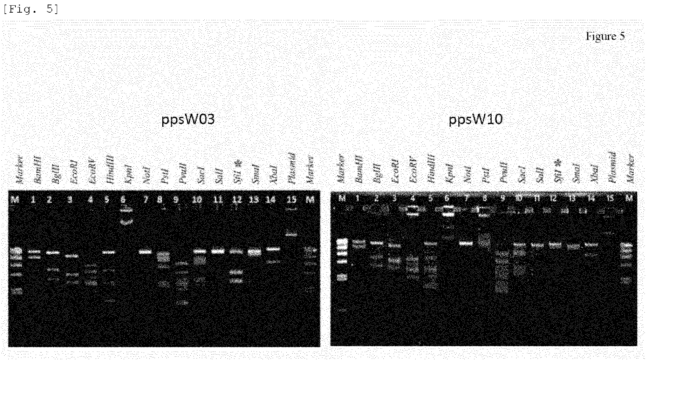

Plipastatin is a cyclic lipopeptide consisting of 10 amino acids produced by Bacillus subtilis, and is biosynthesized by an NRPS. A plipastatin NRPS consists of five enzymes PpsA, PpsB, PpsC, PpsD, and PpsE, as illustrated in FIG. 1, which are involved in the elongation of two, two, two, three, and one specified amino acid, respectively. There are a total of 10 modules between PpsA and PpsE, and the NRPS genes are arranged in the same order on the Bacillus subtilis genome. In the present example, a restriction enzyme SfiI site was introduced into the DNA sequence of the region between the C domain and the A domain of the adjacent module (hereinafter, referred to as C-A linker), which is the boundary of the module. First, among the 10 C-A linker regions present between PpsA and PpsE, the amino acid sequence between the C5 consensus sequence ((IV)GxFVNT(QL)(CA)xR) in the C domain and the A1 consensus sequence (L(TS)YxEL) in the A domain was collected, and multiple alignment analysis was performed using clustralW (FIG. 2). As a result, a portion having diversity in the amino acid sequence was present at a position of 25 amino acids distant from the A1 consensus sequence to the C5 consensus sequence, and thus, attention was paid thereto. The SfiI site 5'-GGCCNNNN/NGGCC-3' (/ may be a cleavage location, and N may be any of A, C, T, and G) spans 13 bases and affects a maximum of 5 consecutive amino acids in the NRPS coding region. In order to minimize the change in the amino acid sequence of the coding region from the wild-type sequence due to the introduction of the SfiI site, while satisfying the point that the overhang sequence generated by each SfiI cleavage required for performing the OGAB method includes the only overhang in consideration of the complementary strand and the point that at least one base of G or C is contained in the overhang three-base sequence, the DNA sequence to be modified was determined according to the following rules (FIG. 3). First, the G base closest to the C domain among the 13 bases of the SfiI site was positioned at the base at position 3 of the codon encoding the most upstream amino acid among the five amino acids that may be potentially affected, thereby increasing the possibility of conversion to a synonymous codon. GCC of bases at positions 2 to 4 resulted in the coding for alanine regardless of the wild-type amino acid sequence. The bases at positions 5 to 7 were made as similar as possible to the wild-type sequence while ensuring the specificity of the overhang sequence to be formed. The bases at positions 8 to 10 were designed so as to encode the same amino acid as that of the wild-type while ensuring the specificity of the overhang sequence to be formed, and even when it was difficult to do so, the base at position 2 of the codon was made the same as that of the wild-type, such that the hydrophobicity and hydrophilicity of the encoded amino acid were not significantly changed. The bases at positions 11 to 13 were adapted to encode alanine regardless of the wild-type amino acid sequence. Based on such design guidelines, as illustrated in FIG. 3 and SEQ ID NOs: 1 to 22, wild-type Bacillus subtilis genomic DNA was amplified by PCR using a primer designed so that the designed SfiI site was positioned at the 5' end of the primer as a template, and cloned into E. coli DH5α by pCR-XL-TOPO kit (Invitrogen) according to the manual, thereby obtaining DNA fragments of pps01 to pps09. For the last module pps10, a primer was designed to amplify to include from the connection portion of pps09 in the C-A linker (upstream) to the putative ρ factor-independent terminator sequence present downstream of the ppsE gene (downstream). In addition, for the pps00 region upstream of pps01, a primer was designed to amplify from the connection portion between pps00 and pps01 so as to include the promoter region of the pps operon. Similarly, the pps00 fragment and the pps10 fragment were cloned into pCR-XL-TOPO, the cloning direction into the vector was confirmed, a fragment containing pps10 obtained by cleaving the plasmid for cloning pps10 with SpeI and XbaI was ligated to the SpeI site of the plasmid for cloning pps00, and then, a clone in which pps00 and pps10 were ligated while maintaining the positional relationship in the genome was selected, thereby constructing pCR-pps00-pps10. The plasmid was partially degraded with the restriction enzyme SfiI, and a 4.3 kb DNA fragment in which pps00 was ligated to pps10 was size-fractionated by low-melting-point agarose gel electrophoresis, and after purification, ligated to DraIII cloning site of pGETS109DraIII of E. coli-Bacillus subtilis shuttle plasmid vector, thereby constructing pGETS109DraIII-pps00-pps10. The plasmid was cleaved with SfiI, and a long DNA fragment was cut out and purified by size fractionation by low-melting-point agarose gel electrophoresis, thereby obtaining a vector DNA fragment that can be used in the OGAB method. The vector DNA fragment and fragments from pps01 to pps09 obtained by cleaving, with SfiI, a plasmid DNA into which DNA fragments from pps01 to pps09 were cloned, were mixed at equimolar concentrations, and assembly was performed by the OGAB method (FIG. 4). After transformation, Bacillus subtilis was smeared on an LB plate containing tetracycline and cultured overnight at 37°C to obtain 57 transformants. After culturing 12 randomly selected colonies in LB medium with tetracycline, the plasmids were purified in small amounts and cleaved with SfiI and other restriction enzymes, and the cleavage pattern was analyzed by agarose gel electrophoresis (FIG. 5). As a result, 5 out of 12 were DNA containing linkage of pps00-pps01-pps02-pps03-pps04-pps05-pps06-pps07-pps08-pps09-pps10. One of them was named ppsW03 (SEQ ID NO: 23).

### Confirmation of production of plipastatin by NRPS introduced with SfiI site

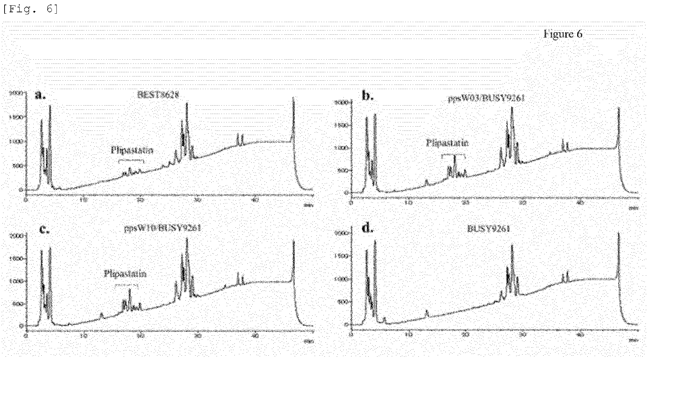

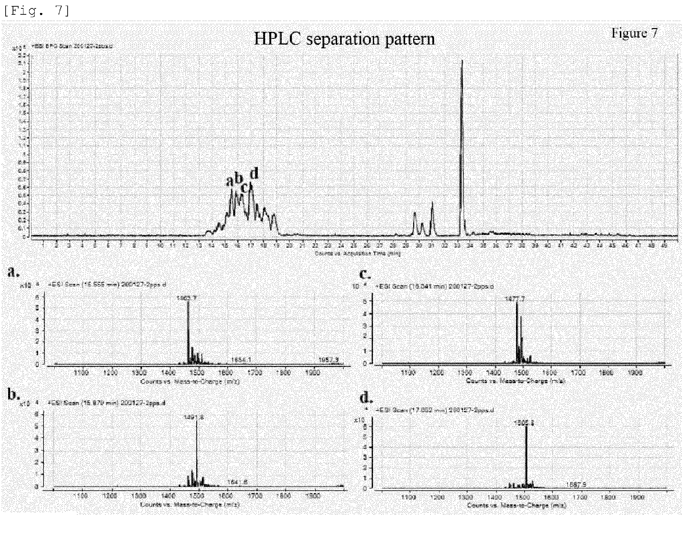

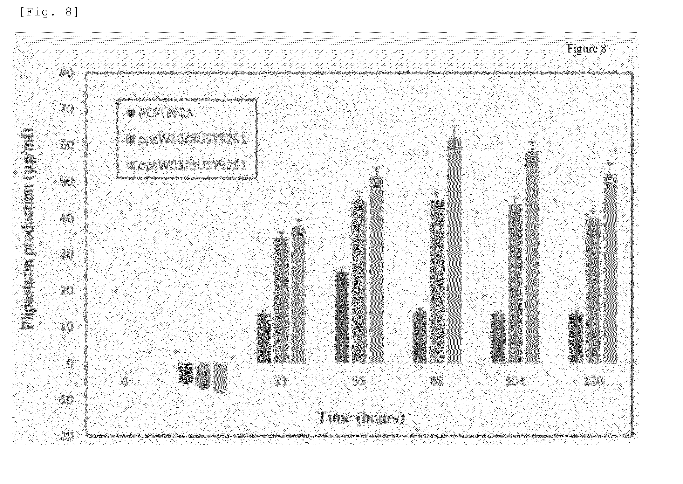

BUSY9261 as an NRPS-producing host was transformed by a Bacillus subtilis competent cell method with ppsW03, and the resulting strain was designated as ppsW03/BUSY9261. The strain was pre-cultured in LB medium containing tetracycline, and then cultured in ACS medium at 30°C for 5 days. The acidic precipitate of the culture solution of the strain was extracted with ethanol and analyzed by analytical HPLC, and a peak was observed at the same retention time as that of plipastatin of the wild-strain BEST8628 strain (FIG. 6). The peak substance was recovered by mass preparative HPLC, and mass spectrometry analysis was performed to confirm that plipastatin was produced (FIG. 7). Plasmid ppsW10 containing a wild-type NRPS with a similar sequence to ppsW03 but no SfiI site was constructed by BReT method (see Tomita, S., Tsuge, K., Kikuchi, Y., Itaya, M., 2004. Targeted isolation of a designated region of the Bacillus subtilisgenome by recombinational transfer. Appl. Environ. Microbiol. 70, 2508-2513) by transforming BEST8628 strain with pGETS109DraIII-pps00-10 cleaved with SfiI (FIG. 5). The ppsW10/BUSY9261 strain into which the resulting product was introduced into the BUSY9261 strain was cultured as a control. As a result, the ppsW03/BUSY9261 strain showed the same amount of plipastatin produced as that of the ppsW10/BUSY9261 strain (FIG. 8). It was confirmed that the introduction of the SfiI site did not affect the amount of plipastatin produced.

### Example 2: Construction of chimeric NRPS

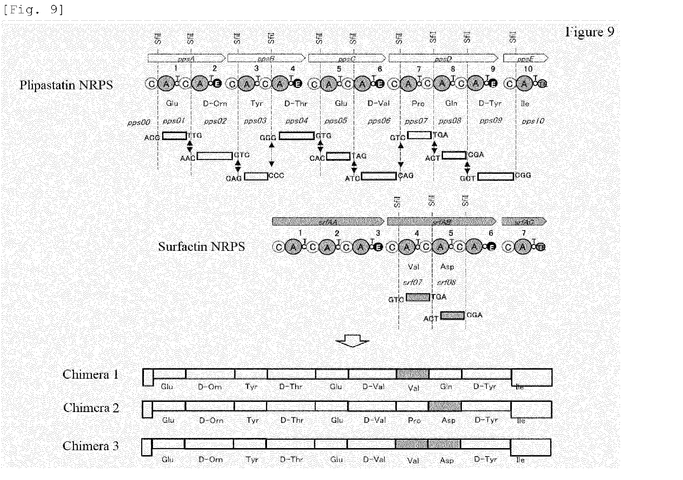

A novel recombinant NRPS gene was constructed by replacing the pps07 fragment encoding the seventh module of the plipastatin NRPS produced in Example 1 and the pps08 fragment encoding the eighth module with DNA fragments of other NRPS-derived modules (FIG. 9). The replacement was performed with a Bacillus subtilis surfactin NRPS module. For the srf07 fragment encoding the fourth module of the surfactin NRPS, the SfiI site was designed so that an overhang sequence identical to pps07 was formed at a position of about 25 amino acids upstream from the A1 consensus sequence, similar to the guidance in Example 1. In addition, similarly, for the srf08 fragment encoding the fifth module of the surfactin NRPS, the SfiI site was designed so that an overhang sequence identical to pps08 was formed at a position of about 25 amino acids upstream from the A1 consensus sequence. As in Example 1, DNA fragments of srf07 and srf08 were amplified using the primers of SEQ ID NOs: 24 to 27 and the genomic DNA of Bacillus subtilis 168 strain as a template, and cloned into pCR-XL-TOPO. The resulting plasmid was cleaved with SfiI to obtain the srf07 fragment and the srf08 fragment.

Three patterns of chimeras 1 to 3 were generated for the chimeric NRPS genes (FIG. 9).
Chimera 1: pps00-pps01-pps02-pps03-pps04-pps05-pps06-srf07-pps08-pps09-pps10 (SEQ ID NO: 28)
Chimera 2: pps00-pps01-pps02-pps03-pps04-pps05-pps06-pps07-srf08-pps09-pps10 (SEQ ID NO: 29)
Chimera 3: pps00-pps01-pps02-pps03-pps04-pps05-pps06-srf07-srf08-pps09-pps10 (SEQ ID NO: 30)

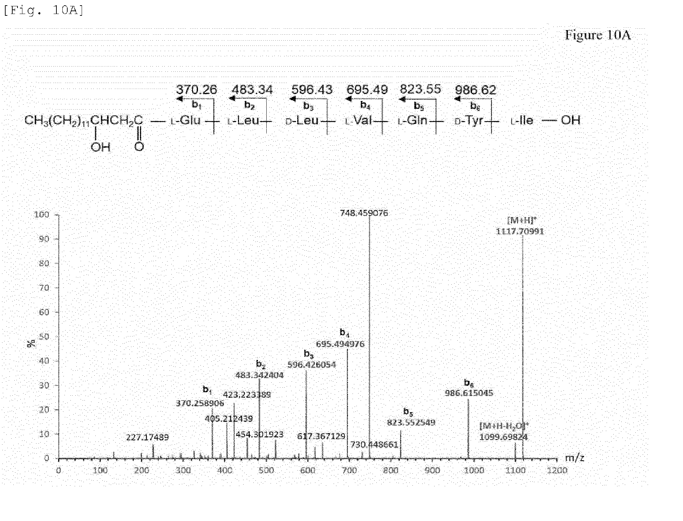

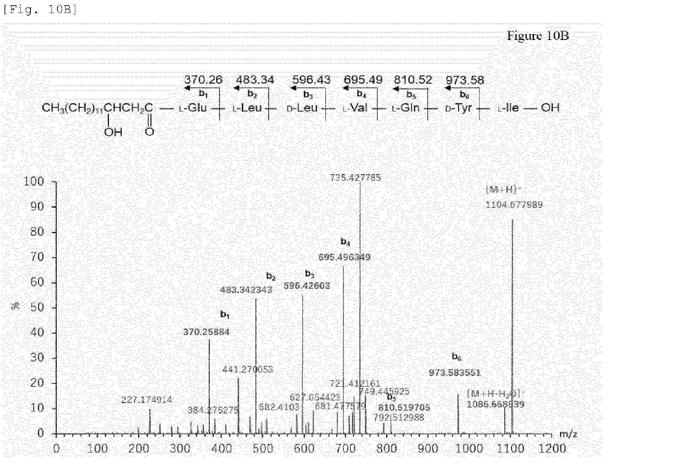

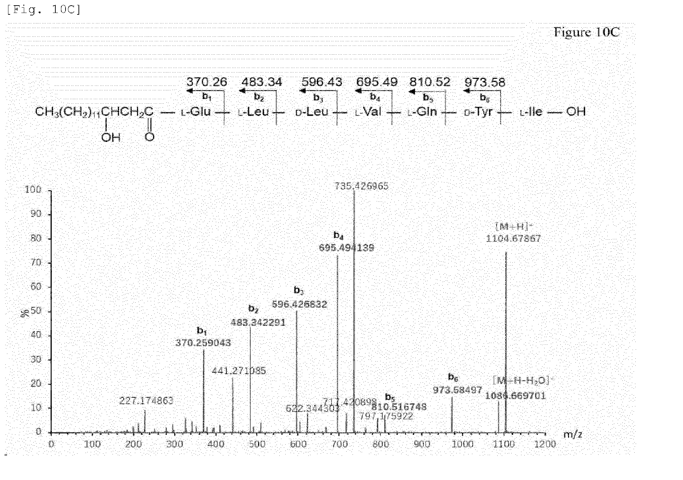

These plasmids were integrated by the OGAB method using Bacillus subtilis host BEST8666 strain. Each of the correctly integrated plasmids was introduced into Bacillus subtilis host BUSY9261 strain deficient in ppsABCDE by a competent cell method, and the resulting strains were designated as BUSY9261/chimera 1, BUSY9261/chimera 2, and BUSY9261/chimera 3, respectively. Each strain was pre-cultured in LB medium, then cultured in ACS medium for 5 days, and analyzed by analytical HPLC, and it was confirmed that these strains produced compounds with mobility different from known plipastatin and surfactin. In order to perform more detailed structural analysis, a new substance was purified by performing mass culture and then performing mass preparative HPLC. As a result of structure determination by LC-MS/MS, it was suggested that, from BUSY9261/chmera 1, a peptide of
· Chimeric peptide 1: β-Hydroxy fatty acid-Glu-Leu-Leu-Val-Gln-Tyr-Ile-OH (SEQ ID NO: 61) was produced,
   and from BUSY9261/chmera 2 and BUSY9261/chmera 3,
· peptides of chimeric peptides 2 and 3: β-Hydroxy fatty acid-Glu-Leu-Leu-Val-Asp-Tyr-Ile-OH (SEQ ID NO: 62)
   were produced (FIG. 10).

The expected peptide products from the NRPS genes encoded by the plasmids of chimeras 1 to 3 were:
· a chimera 1-derived product: β-hydroxy fatty acid-Glu-Orn-Tyr-Thr-Glu-Val-Val-Gln-Tyr-Ile-OH (SEQ ID NO: 63) (alternatively, a lactone ring-formed product between a hydroxyl group on the N-terminal fatty acid chain of the peptide or a hydroxyl group of Tyr closer to the N-terminus and a carboxyl group at the C-terminus);
· a chimera 2-derived product: β-hydroxy fatty acid-Glu-Orn-Tyr-Thr-Glu-Val-Pro-Asp-Tyr-Ile-OH (SEQ ID NO: 64) (alternatively, a lactone ring-formed product between a hydroxyl group on the N-terminal fatty acid chain of the peptide or a hydroxyl group of Tyr closer to the N-terminus and a carboxyl group at the C-terminus); and
· a chimera 3-derived product: β-hydroxy fatty acid-Glu-Orn-Tyr-Thr-Glu-Val-Val-Asp-Tyr-Ile-OH (SEQ ID NO: 65) (alternatively, a lactone ring-formed product between a hydroxyl group on the N-terminal fatty acid chain of the peptide or a hydroxyl group of Tyr closer to the N-terminus and a carboxyl group at the C-terminus).

Unlike the prediction, with the introduced srf07 or srf08 as a boundary, the first half side was a novel peptide derived from a surfactin NRPS and the second half side was a novel peptide derived from a plipastatin NRPS, and the chimeric peptides 2 and 3 were the same peptides. The cause of this is assumed to be a result of integration of the plasmids of chimeras 1 to 3 introduced into BUSY9261 into a wild-type surfactin NRPS gene present in the genomic DNA of the host for some reason, using homology of srf07 or srf08. In any case, a novel peptide was obtained.

### Example 3 (virtual example): Introduction of SfiI recognition sequence into surfactin synthetase

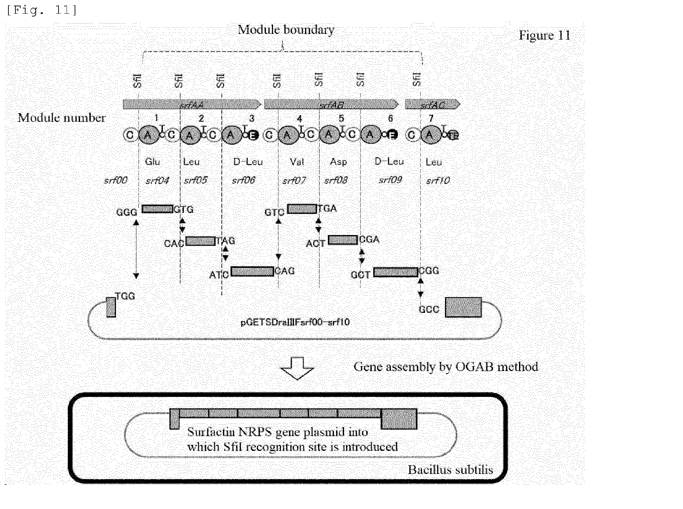

As with the plipastatin NRPS of Example 1, a recognition site for the restriction enzyme SfiI is introduced into the portion of 25 amino acids upstream of the C1 sequence of the consensus sequence of the C domain, as illustrated in FIG. 11. Each block (unit DNA) for the OGAB method is amplified by a PCR method using a primer DNA shown in SEQ ID NOs: 31 to 42. The gene fragment is cloned into pBR-delTypeIIS-3 AarI to prepare a clone of the correct base sequence. srf00 and srf04 to srf10, which are all unit DNAs including srf07 and srf08 of the unit DNA prepared in Example 2, are prepared, and an integrated plasmid in which srf00-srf04-srf05-srf06-srf07-srf08-srf09-srf10 is linked is constructed by BEST9731 strain by the OGAB method as in Example 1 (SEQ ID NO: 43). The plasmid produces surfactin.

### Example 4: Construction of chimeric NRPS library

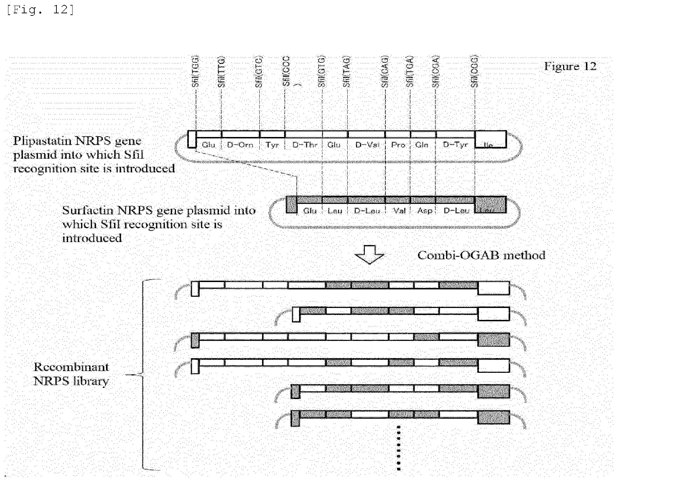

A chimeric plasmid library is constructed by the Combi-OGAB method described in WO 2020/203496 A using the plasmid ppsW03 in which the SfiI site is introduced into the plipastatin NRPS gene produced in Example 1 and the plasmid srf-SfiI in which the SfiI site is introduced into the surfactin NRPS gene produced in Example 3. ppsW03 and srf-SfiI plasmids are purified to high purity by ultracentrifugation, 1 fmol of each plasmid is cleaved with restriction enzyme SfiI, and phenol treatment, butanol treatment, and ethanol precipitation are performed to purify DNA fragments. The obtained DNAs are mixed and ligated in a pseudo tandem repeat form using a 2 × ligation buffer and T4DNA ligase, and the resulting product is introduced by a competent cell method into Bacillus subtilis in which the sfp gene is mounted on BUSY9731 deficient in plipastatin and surfactin NRPSs in terms of amount, such that a chimeric NRPS library can be constructed (FIG. 12).

### Example 5: Introduction of restriction enzyme recognition sites to different sites

In Example 1, the SfiI site was introduced into the 25 amino acids upstream from the C1 consensus sequence of plipastatin NRPS, but in Example 5, unlike this, the restriction enzyme PflMI site is introduced into 25 amino acids downstream from the C3 consensus sequence. There is only one 3'-CCANNNN/NTGG-5' (/ indicates a cleavage location, and N may be any of A, C, T, and G) recognized by PflMI within the plipastatin NRPS. As in Example 1, each unit DNA to which a PflMI site is added is amplified by PCR, cloned into an E. coli plasmid vector, and then cleaved with PflMI, and each unit DNA is purified by low-melting-point agarose gel electrophoresis. These unit DNAs and vector DNAs for OGAB assembly are mixed at equimolar concentrations, and gene assembly is performed by the OGAB method using Bacillus subtilis BEST9731 strain. By introducing the resulting plasmid DNA into BUSY9621 strain, plipastatin is produced by a plipastatin NRPS produced using an NRPS gene with an introduced PflMI site.

### Example 6: Introduction of restriction enzyme site into iturin NRPS

Iturin A produced by Bacillus subtilis RB14 strain is an NRP in which β-amino fatty acid and 7 amino acids are circularly linked and is synthesized by an NRPS encoded by three ORFs of ituA, ituB, and ituC. These three ORFs are contiguous in this order, and there is no restriction enzyme SfiI site inside the gene. In order to introduce a SfiI site into 25 amino acids upstream portion from the C1 consensus sequence of an NRPS of Iturin A, each unit DNA to which a SfiI site is added is amplified by PCR as in Example 1, cloned into an E. coli plasmid vector, and then cleaved with SfiI, and each unit DNA is purified by low-melting-point agarose gel electrophoresis. These unit DNAs and vector DNAs for OGAB assembly are mixed at equimolar concentrations, and gene assembly is performed by the OGAB method using Bacillus subtilis BEST9731 strain. Iturin A is produced by an iturin ANRPS produced using an NRPS gene into which a SfiI site is introduced by introducing the resulting plasmid DNA into BUSY9621 strain.

### (Note)

As described above, the present disclosure is exemplified by the use of preferred embodiments of the present disclosure. It is understood that the scope of the present disclosure should be interpreted solely based on the claims. It is also understood that any patent, any patent application, and any references cited herein should be incorporated herein by reference in the same manner as the contents are specifically described herein. The present application claims priority to Japanese Patent Application No. 2022-121822 filed on July 29, 2022 to the Japanese Patent Office, the entire contents of which are incorporated herein by reference in the same manner as if the entire contents constituted the present application.

### Industrial Applicability

According to the present disclosure, it is possible to efficiently produce a novel NRPS, thereby providing an NRPS that enables efficient NRP production or an NRPS that provides a novel NRP.

### Sequence Listing Free Text

SEQ ID NO: 1: Primer 00-F
   CGACTTGGCCGATGTGGCCCGGGAGACCTGTTTTCCAAGAG
SEQ ID NO: 2: Primer 00-R
   TTTAGGGGCCGCCACGGCCGTCTTGTTAAACTCATACAACAATTTTTG
SEQ ID NO: 3: Primer 01-F
   CAAGACGGCCGTGGCGGCCCCTAAAGCTTTCACCCTGC
SEQ ID NO: 4: Primer 01-R
   TTGAGCGGCCTCAACGGCCGTTTTGTTAAATTCAAAGACTATTTTTTGT T
SEQ ID NO: 5: Primer 02-F
   CAAAACGGCCGTTGAGGCCGCTCAAAAAGATATTCCTTTTCATCGC
SEQ ID NO: 6: Primer 02-R
   GTTCACGGCCGGACGGGCCGTTTCGTTAAACAATTGGATAACCTGTTG
SEQ ID NO: 7: Primer 03-F
   CGAAACGGCCCGTCCGGCCGTGAACAAAACGATACCCCAATTG
SEQ ID NO: 8: Primer 03-R
   TCTCGGGGCCAGGGCGGCCTGCCCCATTTTTTCAAGGAGTAAC
SEQ ID NO: 9: Primer 04-F
   GGGGCAGGCCGCCCTGGCCCCGAGAAATGAAAACATAGTAAGC
SEQ ID NO: 10: Primer 04-R
   TTCAGGGGCCACACTGGCCGTCCTGTTATTTTGAGAAACGATTTTCC
SEQ ID NO: 11: Primer 05-F
   CAGGACGGCCAGTGTGGCCCCTGAAGCCCCAACC
SEQ ID NO: 12: Primer 05-R
   TCTTGGGGCCTCTATGGCCGTACGATTGAGCTCGTGG
SEQ ID NO: 13: Primer 06-F
   TCGTACGGCCATAGAGGCCCCAAGAAATGAAACCATCAGCAG
SEQ ID NO: 14: Primer 06-R
   GGTTTCGGCCACTGGGGCCTGATCTGTGATTTCTCCAATCAGC
SEQ ID NO: 15: Primer 07-F
   AGATCAGGCCCCAGTGGCCGAAACCATCCATGCCATGTTTG
SEQ ID NO: 16: Primer 07-R
   TGAATAGGCCGTCACGGCCAACAGATGGCATGATTCAATGAAATCTC
SEQ ID NO: 17: Primer 08-F
   TCTGTTGGCCGTGACGGCCTATTCAATGAATCAAACGCTCCACTATG
SEQ ID NO: 18: Primer 08-R
   TCCGTAGGCCTTCGCGGCCCCGGTGTTAAACTCATTAAGCAG
SEQ ID NO: 19: Primer 09-F
   CACCGGGGCCGCGAAGGCCTACGGAGTTCAAACAATCAGTCAATTG
SEQ ID NO: 20: Primer 09-R
   CTTTGGGGCCTCCGTGGCCGTATTGTTGAACTGTGTGACAATCTG
SEQ ID NO: 21: Primer 10-F 
CAATACGGCCACGGAGGCCCCAAAGAATCATACAATTATCGATTTATTT
C
SEQ ID NO: 22: Primer 10-R 
AAGCTTGGCCAAGACGGCCATACAAAAAGAAAGACGGACAGAGAATG
GC
SEQ ID NO: 23: ppsW03
   As described in Sequence Listing.
SEQ ID NO: 24: Primer srf07-F
   CAGCACGGCCGCAGAGGCCCCTGAAGGAATGGCTGTTCAC
SEQ ID NO: 25: Primer srf07-R
   GGTTGCGGCCGTCATGGCCGTCATATGTCTTTCTTCTAGCAAAGCAGC
SEQ ID NO: 26: Primer srf08-F 
TATGACGGCCATGACGGCCGCAACCTTTGCAGCACTTTTTGAAAAACA
G
SEQ ID NO: 27: Primer srf08-R
   TGTCGGGGCCGTCGGGGCCTTGCCCTTCCATGCCTCAAGAAGC
SEQ ID NO: 28: Chimera 1
   As described in Sequence Listing.
SEQ ID NO: 29: Chimera 2
   As described in Sequence Listing.
SEQ ID NO: 30: Chimera 3
   As described in Sequence Listing.
SEQ ID NO: 31: Primer srf00-F 
TAGGGCCGATGTGGCCCAAAAATGTCATGAAAGAATCGTTGTAAGACG
CTC
SEQ ID NO: 32: Primer srf00-R
   CTTACGGGCCCCCACGGCCTTACCGCCGGCCCTCG
SEQ ID NO: 33: Primer srf04-F
   CGGTAAGGCCGTGGGGGCCCGTAAGGACATGACGATACCAGAG
SEQ ID NO: 34: Primer srf04-R
   TTTCGGGGCCGCACTGGCCCCCGGGTTAAACTGCTGAATTTGC
SEQ ID NO: 35: Primer srf05-F 
CCCGGGGGCCAGTGCGGCCCCGAAAGATAAAACAATTGTTCAGCTGTT
TGAG
SEQ ID NO: 36: Primer srf05-R
   TGTCGGGGCCGCTAGGGCCTTGCCCTTCCAAGCCTCAAGAAG
SEQ ID NO: 37: Primer srf06-F
   GGGCAAGGCCCTAGCGGCCCCGACAGACAAAACGGTTCATCAGC
SEQ ID NO: 38: Primer srf06-R
   TTCAGGGGCCTCTGCGGCCGTGCTGTTAAACACCTCGACAATTTG
SEQ ID NO: 39: Primer srf09-F
   GGGCAAGGCCCCGACGGCCCCGACAGACAAAACGGTTCATCAG
SEQ ID NO: 40: Primer srf09-R
   TGTTTCGGCCGCCGGGGCCGGCGGGTTTAAGCC
SEQ ID NO: 41: Primer srf10-F
   CCCGCCGGCCCCGGCGGCCGAAACAAAGCCTCTGACGTATTGG
SEQ ID NO: 42: Primer srf10-R 
TAGGGCCAAGAAGGCCTTATGAAACCGTTACGGTTTGTGTATTAAGAA
ATTCGA
SEQ ID NO: 43: srfAA-AC
   As described in Sequence Listing.
SEQ ID NO: 44: A1 core motif sequence
   L(TS)YxEL
SEQ ID NO: 45: A2 core motif sequence
   LKAGxAYL(VL)P(LI)D
SEQ ID NO: 46: A3 core motif sequence
   LAYxxYTSG(ST)TGxPKG
SEQ ID NO: 47: A5 core motif sequence
   NxYGPTE
SEQ ID NO: 48: A6 core motif sequence
   GELxIxGxG(VL)ARGYL
SEQ ID NO: 49: A7 core motif sequence
   Y(RK)TGDL
SEQ ID NO: 50: A8 core motif sequence
   GRxDxQVKIRGxRIELGEIE
SEQ ID NO: 51: A9 core motif sequence
   LPxYM(IV)P
SEQ ID NO: 52: A10 core motif sequence
   NGK(VL)DR
SEQ ID NO: 53: T core motif sequence
   DxFFxxLGG(HD)S(LI)
SEQ ID NO: 54: C1 core motif sequence
   SxAQxR(LM)(WY)xL
SEQ ID NO: 55: C2 core motif sequence
   RHExLRTxF
SEQ ID NO: 56: C3 core motif sequence
   MHHxISDG(WV)S
SEQ ID NO: 57: C4 core motif sequence
   YxD(FY)AVW
SEQ ID NO: 58: C5 core motif sequence
   (IV)GxFVNT(QL)(CA)xR
SEQ ID NO: 59: C6 core motif sequence
   (HN)QD(YV)PFE
SEQ ID NO: 60: C7 core motif sequence
   RDxSRNPL
SEQ ID NO: 61: Chimeric peptide 1
   β-Hydroxy fatty acid-Glu-Leu-Leu-Val-Gln-Tyr-Ile
SEQ ID NO: 62: Chimeric peptides 2 and 3
   β-Hydroxy fatty acid-Glu-Leu-Leu-Val-Asp-Tyr-Ile
SEQ ID NO: 63: Chimera 1 putative peptide
   β-Hydroxy fatty acid-Glu-Orn-Tyr-Thr-Glu-Val-Val-Gln-Tyr-Ile
SEQ ID NO: 64: Chimera 2 putative peptide
   β-Hydroxy fatty acid-Glu-Orn-Tyr-Thr-Glu-Val-Pro-Asp-Tyr-Ile
SEQ ID NO: 65: Chimera 3 putative peptide
   β-Hydroxy fatty acid-Glu-Orn-Tyr-Thr-Glu-Val-Val-Asp-Tyr-Ile

### Sequence Listing

International application No. 093400 under the International Patent Cooperation Treaty Peptide Synthetase Library JP23027837 20230728----00150270152301621357 normal 20230728143752202307181629153720_P1AP101_09_0.xml

## Claims

1. A method for producing a product plasmid containing a nucleic acid sequence encoding a non-ribosomal peptide synthetase (NRPS), the method comprising steps of:
preparing a set of starting plasmids, each of the set of starting plasmids containing a base sequence encoding at least one NRPS module and at least one restriction enzyme recognition sequence;
preparing a mixture of containing a plurality of resulting cleaved nucleic acid fragments by treating the set of starting plasmids with a restriction enzyme;
forming a linked nucleic acid by ligating the plurality of cleaved nucleic acid fragments; and
ringing the linked nucleic acid into contact with a transgenic organism to form a product plasmid.

2. The method according to claim 1, wherein the plurality of cleaved nucleic acid fragments include a set of cleaved nucleic acid fragments containing the same overhang sequence and containing a base sequence encoding a plurality of types of NRPS modules.

3. The method according to claim 1 or 2, wherein the plurality of cleaved nucleic acid fragments include a set of cleaved nucleic acid fragments containing the same overhang sequence and containing the base sequence encoding an NRPS module capturing a plurality of types of amino acids.

4. The method according to any one of claims 1 to 3, wherein each of the cleaved nucleic acid fragments contains an overhang sequence of 3 to 5 bases in length.

5. The method according to any one of claims 1 to 4, wherein in the starting plasmid, the restriction enzyme recognition sequence is located between base sequences encoding adjacent NRPS modules.

6. The method according to any one of claims 1 to 5, wherein in the starting plasmid, the restriction enzyme recognition sequence is located between a C domain coding region and an A domain coding region of the NRPS module.

7. The method according to any one of claims 1 to 6, wherein in the starting plasmid, the restriction enzyme recognition sequence is located in a region between the C domain coding region and the A domain coding region of the NRPS module, and the region does not include the C domain coding region and the A domain coding region.

8. The method according to any one of claims 1 to 7, wherein the at least one restriction enzyme recognition sequence is present in the base sequence encoding an NRPS module.

9. The method according to any one of claims 1 to 8, wherein the at least one restriction enzyme recognition sequence is present outside the base sequence encoding an NRPS module.

10. The method according to any one of claims 1 to 9, wherein the restriction enzyme recognition sequence is a non-native sequence in the NRPS module.

11. The method according to any one of claims 1 to 10, wherein each of the cleaved nucleic acid fragments contains at most one NRPS module.

12. The method according to any one of claims 1 to 11, wherein the restriction enzyme recognition sequence is cleaved at a site different from a restriction enzyme-specific recognition site.

13. The method according to any one of claims 1 to 12, wherein the restriction enzyme recognition sequence includes a recognition sequence for SfiI, BglI, AlwNI, DraIII, PflMI, BstAPI, AarI, BbsI, BsaI, BsmBI, or BspQI.

14. The method according to any one of claims 1 to 13, wherein each of the starting plasmids contains a base sequence encoding 4 to 12 NRPS modules.

15. The method according to any one of claims 1 to 14, wherein in the step of preparing the mixture containing the cleaved nucleic acid fragments, each of the starting plasmids produces cleaved nucleic acid fragments containing overhang sequences that are different from one another.

16. The method according to any one of claims 1 to 15, wherein the mixture containing the cleaved nucleic acid fragments is a solution.

17. The method according to any one of claims 1 to 16, wherein each of the starting plasmids independently contains the same or different selectable marker sequences.

18. The method according to claim 17, wherein the selectable marker sequence includes a drug-resistant marker sequence.

19. The method according to any one of claims 1 to 18, wherein each of the starting plasmids independently contains the same or different origins of replication operable in Bacillus subtilis.

20. The method according to any one of claims 1 to 19, wherein each of the starting plasmids independently contains the same or different promoter regions upstream of the base sequence encoding an NRPS.

21. A product plasmid or plasmid library produced by the method according to any one of claims 1 to 20.

22. An NRPS produced using the product plasmid or plasmid library according to claim 21.

23. An NRP produced using the NRPS according to claim 22.

24. A nucleic acid encoding a non-ribosomal peptide synthetase (NRPS),
the nucleic acid comprising:
a base sequence encoding at least one NRPS module; and
a non-native restriction enzyme recognition sequence for the NRPS module.

25. The nucleic acid according to claim 24, wherein the restriction enzyme recognition sequence is located between the base sequences encoding adjacent NRPS modules.

26. The nucleic acid according to claim 24 or 25, wherein the restriction enzyme recognition sequence is located between a C domain coding region and an A domain coding region of the NRPS module.

27. The nucleic acid according to any one of claims 24 to 26, wherein the restriction enzyme recognition sequence is located in a region between the C domain coding region and the A domain coding region of the NRPS module, and the region does not include the C domain coding region and the A domain coding region.

28. The nucleic acid according to any one of claims 24 to 27, wherein the restriction enzyme recognition sequence is a recognition sequence for a restriction enzyme that recognizes a sequence containing a region in which any type of base is present.

29. The nucleic acid according to any one of claims 24 to 28, wherein the restriction enzyme recognition sequence is a recognition sequence for SfiI.

30. The nucleic acid according to any one of claims 24 to 29, wherein the number of NRPS modules encoded by the nucleic acid is 4 to 12.

31. A method for producing a product nucleic acid containing a nucleic acid sequence encoding a non-ribosomal peptide synthetase (NRPS), the method comprising:
a step of preparing the nucleic acid according to any one of claims 24 to 30 as a starting nucleic acid;
a step of preparing a cleaved nucleic acid fragment by treating the nucleic acid with a restriction enzyme that recognizes the restriction enzyme recognition sequence; and
a step of forming a product nucleic acid that is different from the starting nucleic acid by ligating the cleaved nucleic acid fragments.

32. A product nucleic acid produced by the method according to claim 31.
